# EUROPEAN PATENT SPECIFICATION

(11) **EP 4 255 537 B1**
(45) Date of publication and mention of the grant of the patent: **25.02.2026**
(21) Application number: 21819475.1
(22) Date of filing: 01.12.2021
(51) Int. Cl.: A61M 5/315, A61M 5/20

(54) **ARRANGEMENT FOR A DRUG DELIVERY DEVICE AND DRUG DELIVERY DEVICE**
ANORDNUNG FÜR EINE ARZNEIMITTELABGABEVORRICHTUNG SOWIE ARZNEIMITTELABGABEVORRICHTUNG
ENSEMBLE POUR DISPOSITIF D'ADMINISTRATION DE MÉDICAMENTS ET LEDIT DISPOSITIF

(30) Priority: 02.12.2020 EP 20315476
(43) Date of publication of application: 11.10.2023
(73) Proprietor: Sanofi, 75017 Paris (FR)
(72) Inventor: DASBACH, Uwe, 65926 Frankfurt am Main (DE); KEMP, Thomas Mark, Melbourn, Hertfordshire SG8 6DP (GB); DENYER, Timothy, Melbourn, Hertfordshire SG8 6DP (GB); WILSON, Robbie, Melbourn, Hertfordshire SG8 6DP (GB); ROSIER, Chris, Melbourn, Hertfordshire SG8 6DP (GB)
(74) Representative: Schmidt, Christian
(86) International application number: PCT/EP2021/083843
(87) International publication number: WO 2022/117685

(56) References cited:
- US-A1- 2013 041 327
- US-A1- 2016 106 923

## Description

### Technical field

An arrangement for a drug delivery device is provided. Furthermore, a drug delivery device is provided.

### Background

Administering an injection is a process which presents a number of risks and challenges for users and healthcare professionals, both mental and physical. A drug delivery device may aim to make self-injection easier for patients. A conventional drug delivery device may provide the force for administering the injection by a spring, and trigger button or another mechanism may be used to activate the injection. Drug delivery devices may be single-use or reusable devices.

There remains a need for an improved drug delivery device and an arrangement for a drug delivery device.

US 2016/106923 A1 discloses an injection device with a releasable noise component which generates an audible feedback indicating the end of injection.

### Description

One object to be achieved is to provide an improved arrangement for a drug delivery device. A further object to be achieved is to provide an improved drug delivery device. These objects are achieved, inter alia, by the subject-matter of claims 1 and 15. Advantageous embodiments and further developments are subject of the dependent claims and are also presented in the following description and the figures.

Firstly, the arrangement for the drug delivery device is specified. The arrangement may be a unit of the drug delivery device, e.g. a subassembly of the drug delivery device.

According to at least one embodiment, the arrangement comprises a housing element. The housing element may be hollow and/or elongated. The housing element may be a sleeve, e.g. a cylindrically-shaped sleeve. Particularly, the housing element may be a holder for an energy member such as a drive spring, i.e. an element in which an energy member can be stored. The energy member may be secured to the housing element, e.g. by fixing one end of the drive spring to the housing element.

According to at least one embodiment, the arrangement comprises a plunger rod being arranged axially, i.e. in only one or two opposite axial directions, movable with respect to the housing element. The plunger rod may be cylindrically-shaped.

Here and in the following, if not stated otherwise, a movement of a member or element or feature is to be understood as a movement with respect to the housing element.

The housing element and/or the plunger rod may comprise or consist of plastic. Each of the mentioned elements may be formed in one piece, i.e. be of unitary construction or integrally formed. Each of them may have a main extension direction parallel to a longitudinal axis of the arrangement. The longitudinal axis may run through one or more or every of the mentioned elements, e.g. through the center thereof.

The plunger rod may be received in the housing element so that the housing element circumferentially surrounds, e.g. completely circumferentially surrounds, at least a portion of the plunger rod.

According to at least one embodiment, the arrangement comprises a feedback element being arranged axially movable with respect to the housing element and with respect to the plunger rod. The feedback element may be a piston or a sphere. The feedback element may be axially movable in only one or two opposite axial directions with respect to the housing element and/or the plunger rod. The feedback element may comprise or consist of plastic or metal, e.g. steel. The longitudinal axis may run through the feedback element, e.g. through the center thereof.

According to at least one embodiment, the arrangement comprises a feedback energy member configured to provide energy in order to induce a movement of the feedback element relative to the plunger rod and/or relative to the housing element in a first axial direction. In other words, the feedback energy member may be configured to provide energy to axially move the feedback element relative to the plunger rod and/or relative to the housing element. The feedback energy member may be a spring, e.g. a compression spring, or another component configured to induce a force, e.g. a gas cartridge or an electric motor. The spring may be formed of metal, e.g. steel. The feedback energy member may be secured to the plunger rod or the housing element, e.g. by fixing one end of the spring to the plunger rod or the housing element. The first axial direction may be a distal direction or a proximal direction. The longitudinal axis may run through the feedback energy member, e.g. through the center thereof.

According to at least one embodiment, the arrangement comprises a radially displaceable stop feature. The stop feature may be displaceable in inward and/or outward radial direction. The stop feature is axially and/or rotationally fixed to the plunger rod.

The arrangement may comprise two or more radially displaceable stop features. All features disclosed in connection with the one stop feature are also disclosed for the other stop features. The stop features may be arranged around the longitudinal axis, e.g. with a rotational symmetry with respect to the longitudinal axis.

According to at least one embodiment, the arrangement comprises an impact feature. The impact feature may be axially and/or rotationally and/or radially fixed with respect to the housing element, e.g. may be part of the housing element. The impact feature may be formed at a proximal or distal end of the housing element.

According to at least one embodiment, the arrangement has an initial state, also referred to as locked state or first locked state. The initial state is a state the arrangement can occupy or into which the arrangement can be switched. For example, the initial state is the state in which the arrangement or the drug delivery device is delivered to a user.

According to at least one embodiment, in the initial state, the plunger rod is in a proximal position.

According to at least one embodiment, in the initial state, the feedback element is arranged in a cavity of the plunger rod, i.e. the plunger rod comprises a cavity. The cavity may extend over a main part of the length of the plunger rod. The cavity may be circumferentially surrounded, e.g. completely circumferentially surrounded, by a side wall of the plunger rod. Particularly, in the initial state, the feedback element is received in the plunger rod and may be circumferentially surrounded, e.g. completely circumferentially surrounded, by the plunger rod, e.g. the sidewall thereof.

The plunger rod may be hollow. For example, the plunger rod is hollow cylindrically-shaped. A proximal end and/or a distal end of the plunger rod may be open so that the feedback element can be inserted into the plunger rod or can leave the plunger rod via the respective end. One end of the plunger rod, e.g. the distal end or the proximal end, may be closed so that the feedback element cannot leave the plunger rod via this end.

According to at least one embodiment, in the initial state, the feedback element is limited in its movement relative to the plunger rod in the first axial direction by the stop feature being in a first radial position. In other words, in the initial state, the stop feature is in a first radial position, e.g. is held in the first radial position, in which it limits or prevents a movement of the feedback element in the first axial direction, particularly a movement in the first axial direction beyond the stop feature. For example, in the initial state, the stop feature in the first radial position prevents the feedback element from leaving the plunger rod, i.e. the cavity thereof, by a movement in the first axial direction.

For example, in the initial state, the stop feature being in the first radial position projects into the cavity of the plunger rod so that the feedback element cannot pass the stop feature in the first axial direction. Particularly, in the initial state, the stop feature is located downstream of the feedback element and/or of the feedback energy member along the first axial direction. At least in the initial state, the impact feature may be located downstream of the feedback element and/or of the stop feature and/or of the feedback energy member along the first axial direction.

According to at least one embodiment, the arrangement is configured to be switched from the initial state into a released state.

According to at least one embodiment, in the released state, the plunger rod is moved or moves in a distal direction. Particularly, the plunger rod is moved in the distal direction at least until the plunger rod reaches a feedback position. The plunger rod may be moved manually by a user or may be moved by an energy member of the arrangement.

According to at least one embodiment, the movement of the plunger rod into the feedback position results in the stop feature being radially displaced, i.e. being moved radially. The radial displacement enables a movement of the feedback element relative to the plunger rod in the first axial direction beyond the stop feature. A movement of the stop feature in radial direction may start before or at the time the plunger rod reaches the feedback position. For example, the stop feature is displaced into a second radial position. The second radial position may be a position downstream of the first radial position along the outward radial direction.

When the plunger rod moves from the proximal position into the feedback position, the feedback element and/or the feedback energy member may also move in distal direction, e.g. by the same distance as the plunger rod. For example, the feedback element and/or the feedback energy member is/are axially coupled to the plunger rod during this movement.

According to at least one embodiment, in the released state, particularly at or after the plunger rod has reached the feedback position or the stop feature has been radially displaced, respectively, the feedback element moves relative to the plunger rod and/or relative to the housing element in the first axial direction and beyond the stop feature due to the energy provided by the feedback energy member. Particularly, the feedback element moves in the first axial direction until the feedback element hits against the impact feature. When hitting the impact feature, a tactile and/or audible feedback may be produced.

When moving in the first axial direction relative to the plunger rod, the feedback element may leave the cavity of the plunger rod. For example, when hitting the impact feature, the feedback element is located outside of the plunger rod, e.g. completely outside of the plunger rod, and downstream of the plunger rod along the first axial direction. Particularly, the feedback element is then spaced from the plunger rod in the first axial direction.

After having reached the feedback position, the plunger rod may move further in distal direction until it reaches a distal position. Preferably, the feedback position is closer to the distal position than to the proximal position. Alternatively, the feedback position may be the distal position, i.e. the most distal position, of the plunger rod during its movement in distal direction. This means that after having reached the feedback position, the plunger rod does not move further in distal direction.

In at least one embodiment, the arrangement for a drug delivery device comprises a housing element, a plunger rod being arranged axially movable with respect to the housing element and a feedback element being arranged axially movable with respect to the housing element and with respect to the plunger rod. Moreover, the arrangement comprises a feedback energy member configured to provide energy in order to induce a movement of the feedback element relative to the plunger rod in a first axial direction. The arrangement further comprises a radially displaceable stop feature and an impact feature. The arrangement has an initial state in which the plunger rod is in a proximal position. In the initial state, the feedback element is arranged in a cavity of the plunger rod and the feedback element is limited in its movement relative to the plunger rod in the first axial direction by the stop feature being in a first radial position. The arrangement is configured to be switched from the initial state into a released state in which the plunger rod is moved in a distal direction at least until the plunger rod reaches a feedback position. The movement of the plunger rod into the feedback position results in the stop feature being radially displaced to enable a movement of the feedback element relative to the plunger rod in the first axial direction beyond the stop feature. In the released state, the feedback element moves relative to the plunger rod in the first axial direction and beyond the stop feature due to the energy provided by the feedback energy member until the feedback element hits against the impact feature, thereby producing a tactile and/or audible feedback.

With the feedback element and/or the feedback energy member being arranged in the cavity of the plunger rod, a feedback mechanism is provided which is particularly space-saving. More space for other elements or members, e.g. an energy member, is left.

The drug delivery device and/or the arrangement for the drug delivery device specified herein may be elongated and/or may comprise a longitudinal axis, i.e. a main extension axis. A direction parallel to the longitudinal axis is herein called an axial direction. By way of example, the drug delivery device and/or the arrangement may be cylindrically-shaped.

Furthermore, the drug delivery device and/or the arrangement may comprise a longitudinal end, which may be provide to face or to be pressed against a skin region of a human body. This end is herein called the distal end. A drug or medicament may be supplied via the distal end. The opposing longitudinal end is herein called the proximal end. The proximal end is, during usage, remote from the skin region. The axial direction pointing from the proximal end to the distal end is herein called distal direction. The axial direction pointing from the distal end to the proximal end is herein called proximal direction. A distal end of a member or element of the drug delivery device and/or of the arrangement is herein understood to be the end of the member/element located most distally. Accordingly, the proximal end of a member or element is herein understood to be the end of the element/member located most proximally.

In other words, "distal" is used herein to specify directions, ends or surfaces which are arranged or are to be arranged to face or point towards a dispensing end of the drug delivery device or components thereof and/or point away from, are to be arranged to face away from or face away from the proximal end. On the other hand, "proximal" is herein used to specify directions, ends or surfaces which are arranged or are to be arranged to face away from or point away from the dispensing end and/or from the distal end of the drug delivery device or components thereof. The distal end may be the end closest to the dispensing end and/or furthest away from the proximal end and the proximal end may be the end furthest away from the dispensing end. A proximal surface may face away from the distal end and/or towards the proximal end. A distal surface may face towards the distal end and/or away from the proximal end. The dispensing end may be a needle end where a needle unit is or is to be mounted to the device, for example.

A direction perpendicular to the longitudinal axis and/or intersecting with the longitudinal axis is herein called radial direction. An inward radial direction is a radial direction pointing towards the longitudinal axis. An outward radial direction is a radial direction pointing away from the longitudinal axis.

The terms "angular direction", "azimuthal direction" or "rotational direction" are herein used as synonyms. Such a direction is a direction perpendicular to the longitudinal axis and perpendicular to the radial direction.

An element or member or feature being rotationally, axially or radially fixed with respect to another element or member or feature means that a relative movement in rotational direction or axial direction or radial direction between the two elements/members/features is not possible or prevented.

The terms "protrusion" and "boss" are used as synonyms herein. The term "recess" may particularly stand for an indentation or a cut-out or opening or hole.

According to at least one embodiment, the plunger rod is hollow cylindrically-shaped. Thus, the cavity in the plunger rod may have a cylindrical shape. The length of the cavity, measured along the longitudinal axis, may be at least 50% or at least 75% or at least 80% of the length of the plunger rod.

According to at least one embodiment, in the initial state, the feedback element is completely arranged inside the plunger rod, i.e. in the cavity of the plunger rod. Particularly, the plunger rod projects beyond the feedback element in distal and proximal direction. In other words, in the initial state, the proximal end of the plunger rod is located further proximal than the proximal end of the feedback element and the distal end of the plunger rod is located further distal than the distal end of the feedback element. Likewise, in the initial state, the feedback energy member may be completely arranged in the plunger rod.

According to at least one embodiment, the impact feature is arranged movable with respect to the housing element and/or the plunger rod. For example, the impact feature is arranged rotationally and/or axially movable with respect to the housing element. The impact feature may be part of a movable member of the arrangement, e.g. other than the plunger rod and the feedback element.

According to at least one embodiment, in the released state, the impact feature moves with respect to the housing element and/or the plunger rod. For example, the arrangement is configured such that the impact feature moves with respect to the housing element during movement of the plunger rod from the proximal position towards the feedback position. By way of example, the impact feature moves in the first axial direction, e.g. in proximal direction, and/or rotates. The rotational axis of the impact feature may define or coincide with the longitudinal axis of the arrangement.

According to at least one embodiment, the stop feature is axially and rotationally fixed to the plunger rod. For example, the stop feature may be part of the plunger rod, e.g. integrally formed with the plunger rod.

According to at least one embodiment, in the initial state, the stop feature radially projects into the cavity of the plunger rod. For example, the stop feature is a protrusion projecting into the cavity of the plunger rod. Particularly, the stop feature projects far enough into the cavity in order to limit or block the movement of the feedback element relative to the plunger rod in the first axial direction beyond the stop feature. When trying to move the feedback element in the first axial direction, the feedback element may hit or abut against the stop feature. The stop feature may, particularly, overlap with the plunger rod in axial direction. For example, the stop feature is arranged between the proximal end and the distal end of the plunger rod, e.g. closer to the proximal end than to the distal end.

According to at least one embodiment, the arrangement further comprises an energy member configured to provide energy in order to induce an axial movement of the plunger rod in the distal direction, preferably to drive the plunger rod. The energy member may also be referred to as drive energy member. Particularly, the energy member is different from the feedback energy member.

In other words, the energy member may be configured to provide energy to move the plunger rod in distal direction relative to the housing element. The energy member may be a drive spring, e.g. a torsion drive spring, particularly a spiral torsion spring or clock spring or power spring, or another component configured to induce a movement of the plunger rod, e.g. a gas cartridge or an electric motor. The drive spring may be formed of metal, e.g. steel. The longitudinal axis may run through the center of the drive spring.

The energy member may be received in the housing element and may be circumferentially surrounded, e.g. circumferentially completely surrounded, by the housing element. At least in the initial state, the plunger rod may be received in the energy member and may be circumferentially surrounded, e.g. circumferentially completely surrounded, by the energy member.

According to at least one embodiment, in the initial state, the plunger rod is coupled to the housing element via a lock interface, i.e. via at least one lock interface, which prevents an axial movement of the plunger rod induced by the energy member. In the initial state, the energy member may already induce a force onto the plunger rod. For example, the drive spring is already biased in the initial state.

In other words, in the initial state, a locking mechanism of the arrangement is locked and prevents a movement of the plunger rod in distal direction induced by the energy member.

According to at least one embodiment, in the released state, the lock interface is released so that an axial movement of the plunger rod induced by the energy member is enabled.

According to at least one embodiment, in the released state, the plunger rod moves in distal direction due to the energy provided by the energy member. Particularly, the plunger rod moves from the proximal position in distal direction at least until it reaches the feedback position.

According to at least one embodiment, the arrangement further comprises a transfer member. The transfer member may be arranged rotatably with respect to the housing element. The transfer member may be hollow and/or elongated. The transfer member may be a sleeve. For example, the transfer member is a rotating collar. The transfer member may be configured to be rotated in one or two opposite rotational directions. The rotational axis of the transfer member may define or coincide with the longitudinal axis of the arrangement or the drug delivery device.

The transfer member may comprise or consist of plastic. The plunger rod may be received in the transfer member so that the transfer member circumferentially surrounds, e.g. completely circumferentially surrounds, at least a portion of the plunger rod. The transfer member may be received in the housing element and/or the energy member so that at least a portion of the transfer member is circumferentially surrounded, e.g. completely circumferentially surrounded, by the housing element and/or the energy member.

The impact feature may be axially and/or rotationally and/or radially fixed with respect to the transfer member. For example, the impact feature is part of the transfer member, e.g. integrally formed with the transfer member. By way of example, the impact feature is formed by a surface at an axial end, e.g. the proximal end, of the transfer member, wherein the surface faces towards the feedback element.

According to at least one embodiment, the transfer member and the plunger rod are operatively coupled such that a rotation of the transfer member in a first rotational direction is converted or convertible into an axial movement of the plunger rod in distal direction. The plunger rod and the transfer member may be coupled by a gear, e.g. a threaded interface, transforming a rotational movement of the transfer member into an axial movement of the plunger rod.

According to at least one embodiment, in the released state, the energy member induces a torque onto the transfer member due to which the transfer member rotates in the first rotational direction and thereby forces the plunger rod to move axially in distal direction. In the initial state, the energy member may already induce a torque onto the transfer member. In the initial state, the transfer member may be prevented from a rotation by a rotation-lock interface. i.e. by at least one rotation-lock interface, or by a rotation-locking mechanism, respectively, coupling the transfer member to the housing element. This rotation-lock interface or rotation-locking mechanism may be released in the released state so that the rotation in the first rotational direction induced by the energy member is enabled.

According to at least one embodiment, the plunger rod and the transfer member are operatively coupled via a threaded interface. The threaded interface may be formed directly between the plunger rod and the transfer member. The threaded interface may transform a rotational movement of the transfer member into an axial movement of the plunger rod. For example, the plunger rod comprises a thread engaged with a thread of the transfer member. The thread of the plunger rod may be an external thread, the thread of the transfer member may be an internal thread, or vice versa. The transfer member may be axially secured to the housing element, e.g. via the energy member. For example, one end of the drive spring not fixed to the housing element is fixed to the transfer member. For example, the transfer member is secured to the housing element such that a force necessary for moving the transfer member in one or both axial directions, particularly in the proximal direction, is greater than a force necessary to axially move the plunger rod.

According to at least one embodiment, the plunger rod is rotationally fixed to the housing element, e.g. via a splined interface. This means that the plunger rod does not rotate or is prevented from rotating during movement in axial direction. The splined interface may be formed directly between the plunger rod and the housing element. For example, the plunger rod has a splining element and the housing element has a splining element, e.g. complementarity to and/or mating with the splining element of the plunger rod. The splining elements of the plunger rod and the housing element may engage with each other, e.g. form-lockingly, thereby preventing the rotation of the plunger rod with respect to the housing element. One of the splining elements of the housing element and of the plunger rod may be a groove and the other one of the splining elements of the housing element and the plunger rod may be a protrusion. The protrusion may then engage or project into the groove thereby preventing rotation of the plunger rod. The groove may extend parallel to the longitudinal axis. For example, the groove is formed in the plunger rod and the protrusion is part of the housing element.

Preferably, the splined interface is in close proximity to the threaded interface, e.g. with a distance of at most 1 cm or at most 0.5 cm or at most 0.2 cm. This is beneficial since the torque on the plunger rod is resolved over a short distance reducing the stresses within the plunger rod.

According to at least one embodiment, in the released state, the transfer member rotates by an angle greater than or equal to any one of the following values: 60°, 80°, 120°, 180°, 270°, 360°. For example, in the released state, the transfer member rotates by at least n-times 360°, wherein n is an integer greater than or equal 1. For example, n is one of: 1, 2, 3, 4, 5, 6, 7, 8, 9, 10.

According to at least one embodiment, in the initial state, the displaceable stop feature is held in the first radial position by the transfer member. For example, in the initial state, the transfer member is located radially outwardly with respect to the stop feature and prevents a movement of the stop feature in radial outward direction so that the stop feature cannot leave the first radial position. The stop feature may radially abut against the transfer member in the initial state.

Alternatively, the housing or housing element may hold the stop feature in the first radial position when the arrangement is in the initial state.

According to at least one embodiment, the transfer member comprises a side wall which holds the displaceable stop feature in the first radial position when the arrangement is in the initial state. Particularly, the side wall may be aligned or may overlap with the displaceable stop feature in axial and/or rotational direction. The stop feature may radially abut against the side wall in the initial state, e.g. in radial outward direction.

According to at least one embodiment, a recess is formed in the side wall of the transfer member. The recess may be aligned with the stop feature in axial direction and/or in rotational direction when the plunger rod is in the feedback position so that the displaceable stop feature can radially displace towards or into the recess. For example, the displaceable stop feature in the first radial position is biased towards the transfer member and/or biased in radial outward direction, and automatically displaces towards or into the recess when the plunger rod is in the feedback position.

Additionally or alternatively, the transfer member or the housing or the housing element may comprise a ramp which hits against the displaceable stop feature when the plunger rod moves into the feedback position and thereby forces the stop feature to displace out of the first radial position.

According to at least one embodiment, the arrangement is configured such that, in the released state, the transfer member moves axially with respect to the housing element until it hits an end-stop of the arrangement, e.g. a proximal end-stop. The end-stop may be formed by the housing element or by another element or member axially fixed with respect to the housing element. For example, the transfer member moves by at least 1 mm or at least 5 mm in axial direction, e.g. proximally. Preferably, the transfer member moves axially and/or rotationally during the movement of the plunger rod in distal direction.

According to at least one embodiment, in the released state and after hitting the end-stop, the transfer member continues to rotate. A further axial movement may be prevented by the end-stop. For example, after hitting the end-stop, the transfer member continues to rotate by at least 360°.

According to at least one embodiment, in the released state, the transfer member moves in proximal direction. In this case, the end-stop may be provided in the region of the proximal end of the arrangement.

According to at least one embodiment, the end-stop comprises a friction reduction element. Additionally or alternatively, the proximal end of the transfer member may comprise a friction reduction element.

According to at least one embodiment, a low friction interface is formed between the friction reduction elements of the transfer member and of the end-stop.

According to at least one embodiment, at least one of the friction reduction elements is a tapering protrusion. Particularly, the protrusion tapers in direction of the respective other friction reduction element. The protrusion may have the shape of a cone. For example, the friction reduction element of the end-stop is a tapering protrusion.

According to at least one embodiment, the other one of the friction reduction elements is an indentation. The friction reduction element being the protrusion may protrude into the indentation when the transfer member hits the end-stop. The indentation may be formed by a concave surface at the proximal end of the transfer member.

According to at least one embodiment, the indentation and/or the protrusion are rotationally symmetric, preferably circular symmetric, with respect to the rotational axis of the transfer member and/or the longitudinal axis.

According to at least one embodiment, the energy member is a drive spring, particularly a torsion drive spring, connected to the transfer member at a first connection point and connected to the housing element at a second connection point. The connection of the drive spring to the transfer member and/or the housing element is preferably irreleasable or permanent. That is to say, the connection cannot be released without destroying the connection or the connection is present in every state of the arrangement.

According to at least one embodiment, during axial movement of the transfer member, the first connection point and the second connection point are axially moved with respect to each other. Particularly, the first connection point is moved with respect to the second connection point in proximal direction, when the transfer member moves proximally, e.g. in the released state.

According to at least one embodiment, the feedback energy member is a spring, e.g. a compression spring.

According to at least one embodiment, in the initial state, the feedback energy member is arranged in the cavity of the plunger rod and biases the feedback element in the first axial direction, e.g. in proximal direction. For example, in the initial state, the spring is compressed. For example, in the initial state, the feedback energy member is completely arranged in the plunger rod so that the plunger rod projects beyond the feedback energy member in distal and proximal direction. One end of the spring, e.g. a distal end thereof, may be fixed to the plunger rod, e.g. to a distal end of the plunger rod. At least in the initial state, another end, e.g. the proximal end, of the spring may be in contact or may be fixed to the feedback element.

According to at least one embodiment, in the initial state, the feedback element abuts against the stop feature in the first axial direction. For example, the feedback energy member presses the feedback element against the stop feature in the initial state.

According to at least one embodiment, the stop feature is a protrusion of a displaceable arm. The protrusion my project in radial inward direction. One end of the displaceable arm may be rotationally and/or axially and/or radially fixed to a member of the arrangement other than the feedback element, e.g. to the plunger rod or to the transfer member or to the housing element. The other arm may be free and may be displaceable in radial direction. The protrusion forming the stop feature may be located closer to the free end than to the fixed end.

The displaceable arm may be a flexible arm, particularly a resilient arm. The displaceable arm is part of the arrangement. The displaceable arm may be part of the plunger rod or the transfer member or the housing element, e.g. integrally formed with the respective element or member.

According to at least one embodiment, the displaceable arm is oriented axially. This means, a main extension direction of the displaceable arm is along an axial direction. The displaceable arm may be elongated.

According to at least one embodiment, the stop feature is arranged in the region of the proximal end of the plunger rod. Particularly, the stop feature may be located closer to the proximal end of the plunger rod than to the distal end of the plunger rod.

According to at least one embodiment, at least one of the stop feature and the feedback element comprises a slide feature against which the other element, i.e. either the stop feature or the feedback element, can abut and along which the other element can slide for displacing the stop feature in radial direction. The slide feature may be a beveled surface. The beveled surface may be tilted with respect to the axial direction and/or the radial direction. For example, an angle between the beveled surface and the axial direction and/or the radial direction is at least 10° and at most 80°. The beveled surface may be oriented parallel to the rotational direction.

The slide feature may be configured such that when the feedback element is pressed against the stop feature, a part of the force pressing the feedback element against the stop feature is converted into a force pointing in radial direction, e.g. in radial outward direction. This may bias the stop feature in the corresponding radial direction. For example, the feedback element and the stop feature abut against each other at the beveled surface.

According to at least one embodiment, in the initial state, the displaceable stop feature being in the first radial position is biased in a radial direction, e.g. in the outward radial direction. The stop feature may be biased in radial direction by the feedback energy member. For example, the feedback element abutting against the stop feature and being pressed against the stop feature due to the feedback energy member may bias the stop feature in the radial direction.

According to at least one embodiment, when the plunger rod reaches the feedback position, the displaceable stop feature automatically moves out of the first radial position into the second radial position.

According to at least one embodiment, in the released state, the distance the feedback element moves in the first axial direction induced by the feedback energy member is at least the distance between the proximal position and the feedback position of the plunger rod. Preferably, the distance the feedback element moves in the released state is larger than the distance between the proximal position and the feedback position. Particularly, the feedback element leaves the cavity of the plunger rod when moving in the first axial direction. For example, the feedback element moves out of the plunger rod, passes an axial end of the plunger rod, e.g. the proximal end of the plunger rod, and moves further in the first axial direction until it hits the impact feature.

According to at least one embodiment, the feedback element hitting the impact feature creates an audible feedback of at least 20 dB or at least 30 dB or at least 40 dB or at least 60 dB. Particularly, the feedback element hitting the impact feature creates a feedback (tactile and/or audible) which is noticeable by a user of the arrangement or the drug delivery device. The tactile and/or audible feedback created when the feedback element hits the impact feature may me greater or louder than any other feedback or mechanism of the arrangement or the drug delivery device.

Next, the drug delivery device is specified. Particularly, the drug delivery device comprises the arrangement according to any one of the described embodiments. Thus, all features described in connection with the arrangement are also disclosed for the drug delivery device and vice versa. The drug delivery device may be an auto-injector.

According to at least one embodiment, the drug delivery device comprises the arrangement according to any one of the above-described embodiments.

According to at least one embodiment, the drug delivery device comprises a housing. The housing element may be fixed to the housing or integrated in the housing. The housing is preferably axially and rotationally, preferably also radially, fixed with respect to the housing element. The housing element may be part of the housing, e.g. integrally formed with the housing, or may be a separate element. The housing may comprise or consist of plastic and/or may be formed in one piece. The housing may be hollow and/or elongated and/or hollow cylindrically-shaped. The housing may be a sleeve. The housing may be configured to hold or receive a medicament container, e.g. a syringe. The housing may be configured to hold the medicament container such that it is axially and/or rotationally and/or radially fixed with respect to the housing. The housing element and/or the energy member and/or the plunger rod and/or the transfer member may be received in the housing, i.e. circumferentially surrounded by the housing.

The impact feature may be axially and/or rotationally and/or radially fixed to the housing, e.g. may be part of the housing. The impact feature may be formed at a proximal or distal end of the housing. The displaceable stop feature may be axially and/or rotationally fixed with respect to the housing, e.g. may be part of the housing.

According to at least one embodiment, the drug delivery device comprises the medicament container. The medicament container may comprise a needle. The arrangement and the medicament container may be received in the housing, i.e. circumferentially surrounded by the housing. The needle may form the distal end of the medicament container, the medicament container may be located distally with respect to the transfer member and/or the plunger rod and/or the energy member, especially in the initial state. The medicament container may be arranged axially and/or rotationally and/or radially fixed with respect to the housing, i.e. it is not moved with respect to the housing during the intended usage of the drug delivery device. The medicament container may be a syringe, e.g. a pre-filled syringe. An end of the container opposite the needle may be sealingly closed by a movable member, e.g. a stopper or piston. The medicament container may comprise a drug or medicament, e.g. a liquid drug or medicament. The drug delivery device may be configured to empty the medicament container when released. In other words, the medicament container may comprise medicament in an amount sufficient for just one drug delivery operation. The drug delivery operation may be performed when the drug delivery device or the arrangement, respectively, has been switched into the released state. The drug delivery device may be a single use device and/or a disposable device.

According to at least one embodiment, the drug delivery device comprises a needle shroud telescopically coupled to the housing and axially movable with respect to the housing between an extended position, e.g. a position in which the needle is covered by the needle shroud, and a retracted position, e.g. a position in which the needle is exposed. In the retracted position, the needle can be pierced into tissue of a body. The needle shroud may be rotationally fixed to the housing.

For example, in the initial state, the needle shroud is in its extended position. For example, when moving the needle shroud from its extended position to the retracted position, the needle shroud is moved in the proximal direction. The movement into the retracted position may release the locking mechanism or the lock interface, respectively, and may switch the arrangement/drug delivery device from the initial state into the released state.

According to at least one embodiment, the drug delivery device comprises a shroud spring. The shroud spring may be coupled to the needle shroud and the housing and/or housing element. The shroud spring may be configured such that it induces a restoring force acting in distal direction on the needle shroud when the needle shroud is moved from the extended position towards the retracted position.

According to at least one embodiment, the medicament container comprises a stopper. The stopper may seal the medicament container in proximal direction. In the released state of the drug delivery device, a distal end of the plunger rod may abut against the stopper and may, driven by the energy member, push the stopper in distal direction. The movement of the stopper in distal direction may result in the drug in the medicament container to be pressed through the needle out of the drug delivery device.

According to at least one embodiment, in the initial state, the plunger rod is axially spaced from the stopper. Thus, in the released state, the plunger rod first moves in distal direction before it hits the stopper and then it pushes the stopper in distal direction. The axial movement of the transfer member preferably starts simultaneously with the axial movement of the plunger rod. Alternatively, the axial movement of the transfer member may only start with or after the plunger rod hits the stopper.

According to at least one embodiment, the movement of the stopper may start with a delay compared to the start of the movement of the transfer member and/or the plunger rod. For example, the transfer member first moves in the first rotational direction and/or axially for a certain distance before the stopper starts to move.

The drug delivery device may be used as follows: First, the drug delivery device or the arrangement thereof, respectively, is in the initial state. Then, the distal end of the drug delivery device is pressed against a skin region of a body, e.g. a human body. At this state, the distal end of the drug delivery device may be formed by a distal end of the needle shroud. This forces the needle shroud to move from the extended position into the retracted position. This movement biases the shroud spring and the biased shroud spring biases the needle shroud in distal direction with respect to the housing. In the retracted position, the locking mechanism is released and the drug delivery device switches from the initial state into the released state. In the released state, the drug is delivered, for example injected into the tissue of the body. This happens due to the movement of the plunger rod in distal direction. At or near the end of the drug delivery, the plunger rod reaches the feedback position. This results in a radial displacement of the stop feature and a movement of the feedback element, driven by the feedback energy member, in the first axial direction, e.g. the proximal direction, until the feedback element hits the impact feature. This creates an audible and/or tactile feedback indicating the user the end of dose injection. The user may now remove the distal end of the drug delivery device from the skin. The shroud spring forces the needle shroud to move in distal direction, e.g. back into the extended position.

Hereinafter, the arrangement for a drug delivery device and the drug delivery device described herein will be explained in more detail with reference to drawings on the basis of exemplary embodiments. Same reference signs indicate same elements in the individual figures. However, the size ratios involved are not necessarily to scale, individual elements may rather be illustrated with an exaggerated size for a better understanding.

### Brief description of the drawings

Figures 1 to 6 show a first exemplary embodiment of the drug delivery device in different views,
Figure 7 to 12 show different positions during usage of the drug delivery device according to the first exemplary embodiment,
Figure 13 shows the drug delivery device according to the first exemplary embodiment in an exploded view,
Figures 14 to 16 show subassemblies of the drug delivery device according to the first exemplary embodiment in more detail,
Figures 17 to 22 show a second exemplary embodiment of the drug delivery device in different views,
Figures 23 and 24 show subassemblies of the drug delivery device according to the second exemplary embodiment in exploded views,
Figures 25 to 27 show a part or arrangement of the drug delivery device according to the first and second exemplary embodiment in different positions during usage for illustrating an exemplary embodiment of a drive mechanism,
Figures 28 to 33 show sections of the drug delivery device according to the first and second exemplary embodiment in different positions during usage for illustrating an exemplary embodiment of a first locking mechanism and the release of the first locking mechanism,
Figures 34 to 38 show sections of the drug delivery device according the first and second exemplary embodiment in different positions during usage for illustrating a first exemplary embodiment of a third locking mechanism,
Figures 39 and 40 show sections of the drug delivery device in different positions during usage for illustrating a second exemplary embodiment of the third locking mechanism,
Figures 41 and 42 show sections of the drug delivery device according to the first and second exemplary embodiment in different positions during usage for illustrating an exemplary embodiment of a drop protection mechanism,
Figure 43 shows the different subassemblies of the drug delivery device according to the first exemplary embodiment and a step during assembling a drug delivery device,
Figures 44 to 46 show sections of the front subassembly of the drug delivery device according to the first exemplary embodiment,
Figures 47, 48 and 50 to 53 show different positions in an exemplary embodiment of a method for assembling the drug delivery device according to the first exemplary embodiment,
Figure 49 shows an isolated drive spring holder of the drug delivery according to the first and second exemplary embodiments,
Figures 54 to 56 show an exemplary embodiment of a feedback mechanism in different positions,
Figures 57 to 62 show a third exemplary embodiment of a drug delivery device in different views,
Figure 63 shows the drug delivery device according to the third exemplary embodiment after usage,
Figure 64 shows different subassemblies of the drug delivery device according to the third exemplary embodiment,
Figures 65 and 66 show the subassemblies of the drug delivery device according to the third exemplary embodiment in exploded views,
Figures 67 to 70 show sections of the drug delivery device according to the third exemplary embodiment in different positions during usage for illustrating a locking mechanism,
Figures 71 to 73 show different positions during assembling the drug delivery device according to the third exemplary embodiment.

### Description of exemplary embodiments

### 1. First exemplary embodiment of a drug delivery device

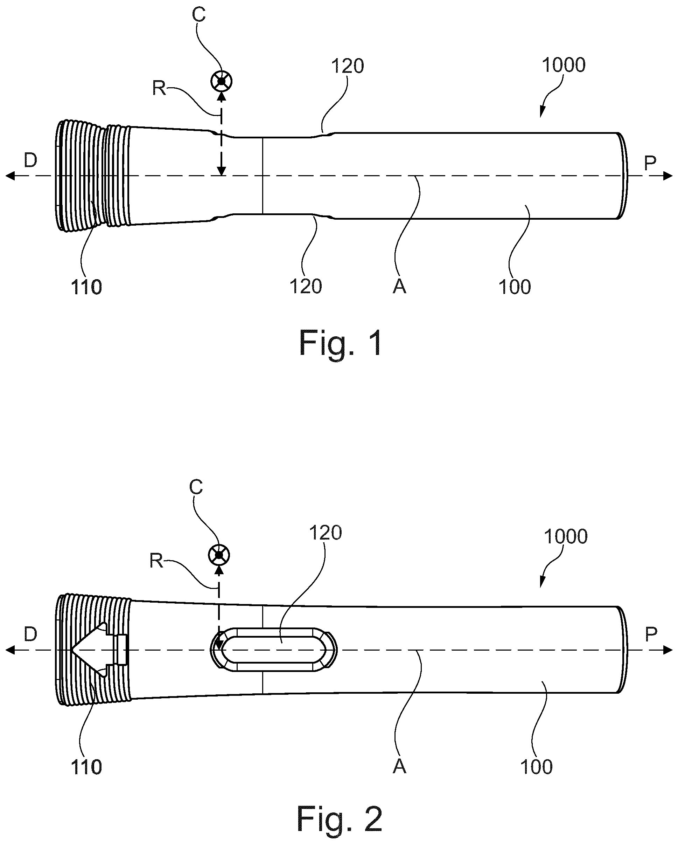

Figures 1 and 2 show side views of a first exemplary embodiment of the drug delivery device 1000. Figure 1 shows a first view of the drug delivery device 1000 and figure 2 shows a second view in which the device 1000 is rotated by 90° around a longitudinal axis A compared to the first view.

Figures 1 and 2 also indicate the coordinate system used herein for specifying positions of members or elements or features. The distal direction D and proximal direction P run parallel to the longitudinal axis A. The longitudinal axis A is a main extension axis of the device 1000. The radial direction R is a direction perpendicular to the longitudinal axis A and intersecting with the longitudinal axis A. The azimuthal direction C, also referred to as angular direction or rotational direction, is a direction perpendicular to the radial direction R and to the longitudinal axis A. The different directions and axes will not be indicated in every of the following figures in order to increase the clarity of the figures.

The drug delivery device 1000 according to the first exemplary embodiment is an auto-injector. The auto-injector 1000 comprises a housing 100. A cap 110 is removably attached or coupled to the housing 100 at a distal end of the housing 100. The housing 100 may be formed in one piece and may extend from the cap 110 to the proximal end of the auto-injector 1000. The housing 100 is a cylindrically-shaped sleeve.

As can be further seen in figures 1 and 2, the housing 100 comprises windows 120 through which a medicament container inside the housing 100 can be investigated. For example, the fill level of the drug inside the medicament container or the advancement of a stopper in the medicament container or the drug clarity or the degradation of the drug can be observed through the windows 120.

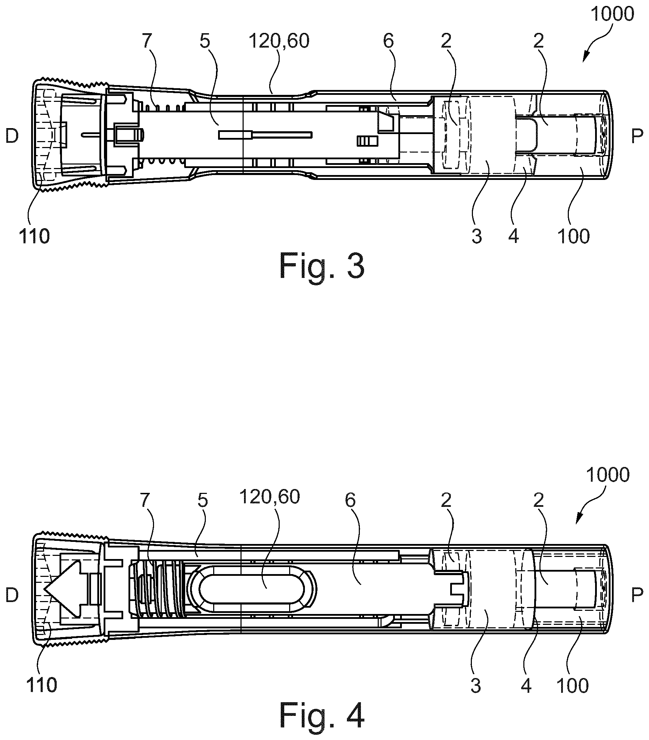

Figures 3 and 4 show the auto-injector 1000 in the same views as in figures 1 and 2, but now the cap 110 and the housing 100 are indicated semi-transparent so that further details of the auto-injector 1000, which are normally completely surrounded and hidden by the housing 100 and the cap 110, are visible. It can be seen that the auto-injector 1000 further comprises a transfer member 2, also referred to as moveable member 2 or drive member 2, respectively, in the form of a rotating collar 2, an energy member 3 in the form of a torsion drive spring 3, particularly a spiral torsion drive spring (also commonly referred to as clock spring or power spring), and a housing element 4 in the form of a drive spring holder 4.

The drive spring holder 4 is fixed to the housing 100 so that the drive spring holder 4 can neither be rotated nor axially nor radially moved with respect to the housing 100. For example, the drive spring holder 4 is fixed with help of clips (not shown) to the housing 100. Alternatively, the drive spring holder 4 may be part of the housing 100, e.g. integrally formed with the housing 100. The drive spring holder 4 is received in the housing 100. The housing 100 circumferentially completely surrounds the drive spring holder 4.

The torsion drive spring 3 is connected to the drive spring holder 4 at a connection point. At a further connection point, the torsion drive spring 3 is connected to the rotating collar 2. The connection points are not visible in the figures. The rotating collar 2 is arranged axially and rotationally movable with respect to the drive spring holder 4. The torsion drive spring 3 circumferentially surrounds a portion of the rotating collar 2. When the torsion drive spring 3 is biased, it induces a torque onto the rotating collar 2. This torque results in a rotation of the rotating collar 2 with respect to the drive spring holder 4, if the rotating collar 2 is not prevented from rotating by a locking mechanism (see explanations further down below). The rotational axis of the rotating collar 2 may define or coincide with the longitudinal axis A.

The auto-injector 1000 further comprises a release member 5 or protection member 5, respectively, in the form of a needle shroud 5 and a medicament container holder 6 in the form of a syringe holder 6. The syringe holder 6 may be axially and preferably also rotationally fixed with respect to the housing 100. The syringe holder 6 is configured to hold a syringe. The syringe holder 6 comprises windows 60 which overlap/ are aligned with the windows 120 in the housing 100. In this way, the syringe or medicament container can be observed through the windows 60, 120.

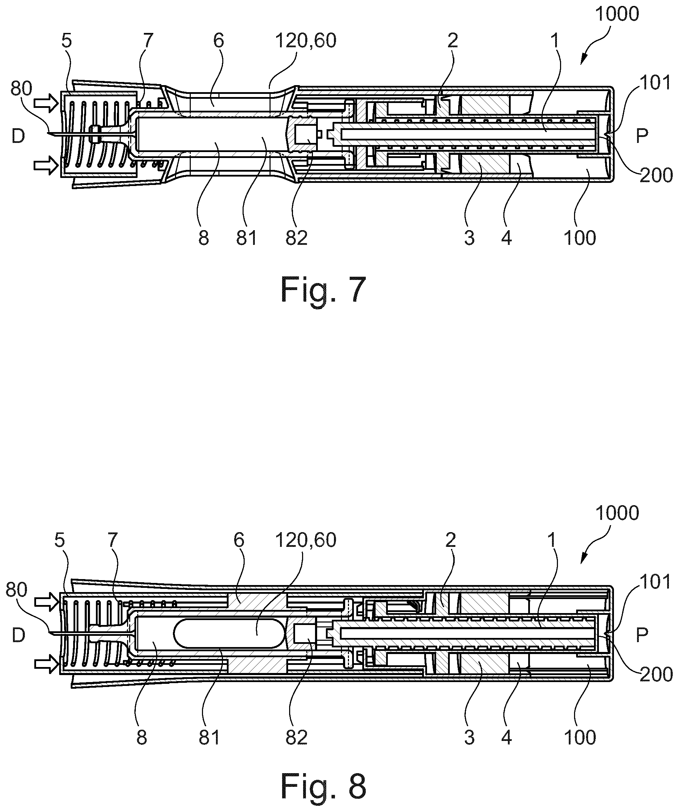

The needle shroud 5 is arranged axially movable with respect to and telescopically coupled to the housing 100 or the drive spring holder 4, respectively. Particularly, the needle shroud 5 can be moved from an extended position, which is the position shown in figure 3 and 4, in the proximal direction P, into a retracted position (see figures 7 and 8). This will be explained in more detail further below.

The needle shroud 5 and the syringe holder 6 are moveably coupled to each other via a shroud spring 7. One end of the shroud spring 7 is connected to the syringe holder 6 and the other end of the shroud spring 7 is connected to the needle shroud 5. The coupling is such that a movement of the needle shroud 5 in the proximal direction P with respect to the syringe holder 6 results in a compression of the shroud spring 7 inducing a force onto the needle shroud 5 pointing in distal direction D.

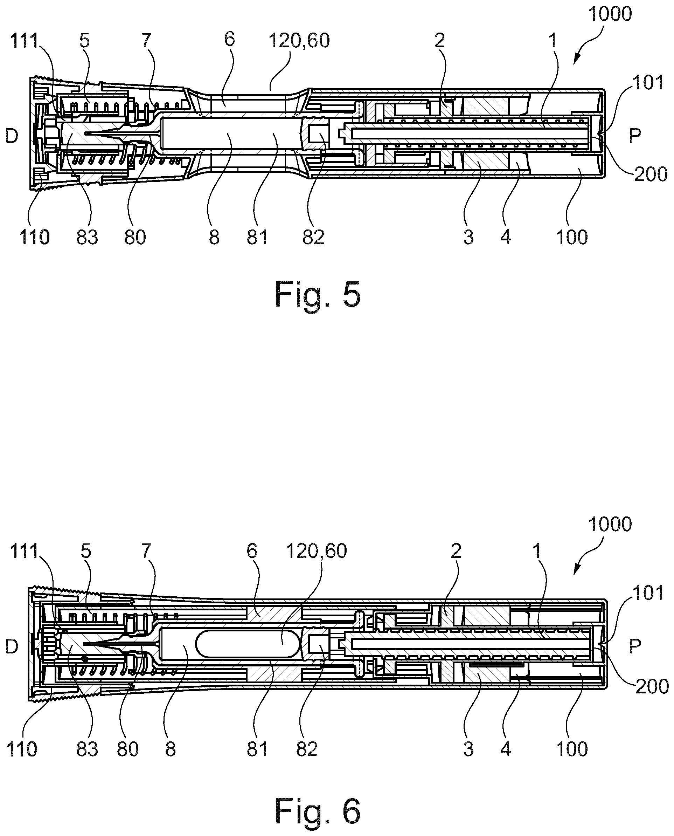

Figures 5 and 6 show the auto-injector 1000 in two cross-sectional views, the views again being rotated by 90° with respect to each other around the longitudinal axis. The cutting plane comprises the longitudinal axis A. In this view, it can be seen that the auto-injector 1000 further comprises a plunger rod 1. The plunger rod 1 is to a main part arranged inside the rotating collar 2 and is circumferentially surrounded by the rotating collar 2. Only a small portion of the plunger rod 1 (less than 50 % of its length) projects out of the rotating collar 2 in distal direction D. In proximal direction P, the rotating collar 2 is closed and the plunger rod 1 does not project beyond the proximal end of the rotating collar 2. The plunger rod 1 is longer, measured along the longitudinal axis A, than the rotating collar 2.

The housing 100, the housing element 4, the plunger rod 1, the rotating collar 2, the needle shroud 5, the syringe holder 6 and the cap 110 may all comprise or consist of plastic. All these members may each be formed in one piece. The drive spring 3 and the shroud spring 7 may comprise or consist of a metal, e.g. steel.

It can be seen in figures 5 and 6 that a medicament container 8, in the present case a syringe 8, is arranged in the syringe holder 6. This syringe 8 may be arranged axially and/or rotationally and/or radially fixed with respect to the syringe holder 6 and/or with respect to the housing 100. The syringe 8 comprises a cartridge 81 filled with a drug, a needle 80 and a stopper 82. The needle 80 is arranged at a distal end of the syringe 8. The stopper 82 seals the cartridge 81 in proximal direction P. When moving the stopper 82 in the distal direction D, the drug stored in the cartridge 81 is pressed out of the syringe 8 through the needle 80.

In figures 5 and 6 it can be further seen that the needle 80 is covered by a needle shield 83 which encapsulates the needle 80 and projects beyond the needle 80 in distal direction D. The needle shield 83 may be formed of a rubber material. The cap 110 is connected to a grabber 111. The grabber 111 is retained within the cap 110 with a one or more bosses. The grabber 111 is coupled with the needle shield 83. The grabber 111 may be formed of a metal and may comprise barbs, which engage into the material of the needle shield 83.

When the cap 110 is removed from the housing 100, the grabber 111 pulls of the needle shield 83 from the needle 80. Afterwards, the needle 80 is circumferentially only surrounded by the retractable needle shroud 5.

Figures 7 and 8 show the auto-injector 1000 in the two cross-sectional views during usage. A first position during usage is shown in which the cap 110, the grabber 111 and the needle shield 83 have been removed from the housing 100. The needle shroud 5 projects from the housing 100 in distal direction D.

In the position of figures 7 and 8, the distal end of the auto-injector 1000 formed by the needle shroud 5 may be pressed against a body, e.g. a human body. As a consequence of that, the needle shroud 5 moves from its extended position in the proximal direction P with respect to the housing 100. This results in the needle 80 being exposed and projecting in distal direction D beyond the needle shroud 5 so that it can now pierce or is already pierced into the tissue of the body.

In the position of figures 7 and 8, the auto-injector 1000 is still in a first locked state, also referred to as pre-released state or initial state, (like in the previous figures), in which the torsion drive spring 3 is biased and induces a torque onto the rotating collar 2. A first locking mechanism (also referred to as first rotation-locking mechanism), however, prevents the rotating collar 2 from a rotational movement. The first locking mechanism will be explained in more detail further below.

In the first locked state, a proximal end of the rotating collar 2 is axially spaced from a proximal end-stop of the housing 100. This allows an axial movement of the rotating collar 2 in proximal direction P. Moreover, in the first locked state, a distal end of the plunger rod 1 is axially spaced from the stopper 82 of the syringe 8. Thus, the plunger rod 1 can axially move in the distal direction D for a predetermined distance before hitting the stopper 82.

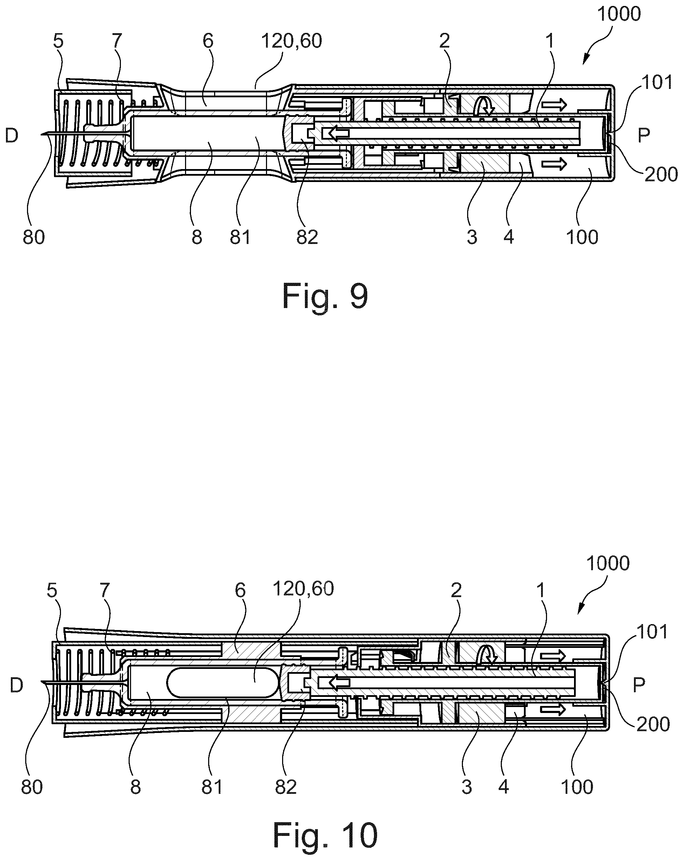

Figures 9 and 10 show the two cross-sectional views of the auto-injector 1000 in a second position during usage. The auto-injector 1000 is now in a released state. The needle shroud 5 has been further moved in the proximal direction P into a retracted position. This has released the first locking mechanism so that the rotating collar 2 was no longer prevented from rotating. The torque induced by the torsion drive spring 3 onto the rotating collar 2 forces the rotating collar 2 to rotate in a first rotational direction (clockwise or counterclockwise). A drive mechanism, which will be explained in more detail further below, has converted the rotation of the rotating collar 2 into an axial movement of the plunger rod 1 in the distal direction D. After having moved the predetermined distance in the distal direction D, the plunger rod 1 has hit the stopper 82 of the syringe 8 and can now push the stopper 82 in distal direction D which results in the drug in the cartridge 81 being pressed out through the needle 80 into the tissue.

As indicated in figures 9 and 10, the rotating collar 2 does not only rotate but also moves in proximal direction P until the proximal end of the rotating collar 2 hits the proximal end-stop of the housing 100. The end-stop comprises is a protrusion 101 which tapers in distal direction D. The protrusion 101 may be a cone. The proximal end of the rotating collar 2 comprises an indentation 200. For example, the surface of the proximal end of the rotating collar 2 is concavely shaped. The protrusion 101 can penetrate into the indentation 200 when the proximal end of the rotating collar 2 hits the end-stop of the housing 100. The protrusion 101 and the indentation 200 may each be designed rotationally symmetric or circular symmetric with respect to the rotational axis of the rotating collar 2. In this way, a low friction interface is formed between the housing 100 and the rotating collar 2 so that a low friction rotation of the rotating collar 2 is enabled also when the proximal end of the rotating collar 2 abuts against the housing 100. Particularly, the radius at which the friction between the rotating collar 2 and the end-stop acts is approaching zero or is zero, therefore the resulting torque from the friction also tends to zero significantly reducing losses allowing a reduced spring force and/or enhance injection performance.

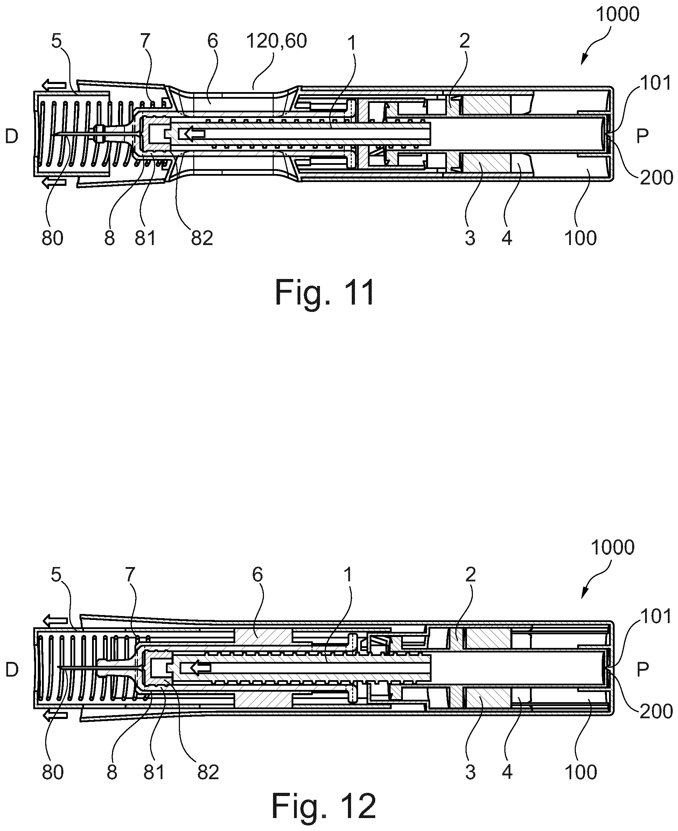

Figures 11 and 12 show two cross-sectional views of the auto-injector 100 in a third position during usage. The torsion drive spring 3 has further induced torque onto the rotating collar 2, which, although abutting against the end-stop of the housing 100, has further rotated and has thereby forced the plunger rod 1 to move further in distal direction D. The plunger rod 1 has pushed the stopper 82 further in distal direction D so that a predetermined dose of the drug was supplied through the needle 80, e.g. into the tissue. Between the described first and third position, the rotating collar 2 has, e.g., rotated several times around its rotational axis.

In figures 11 and 12, the auto-injector 1000 is in a third locked state or post-released state, in which the needle shroud 5 is again in its extended position so that it circumferentially surrounds the needle 80 and so that the needle 80 does no longer distally project beyond the needle shroud 5. The movement of the needle shroud 5 in the extended position happens automatically due to the force induced by the shroud spring 7 which has been compressed when moving the needle shroud 5 out of the extended position towards the retracted position.

In the third locked state of the auto-injector 1000 shown in figures 11 and 12, the needle shroud 5 cannot be moved back into the retracted position due to a third locking mechanism which will be explained in more detail further below.

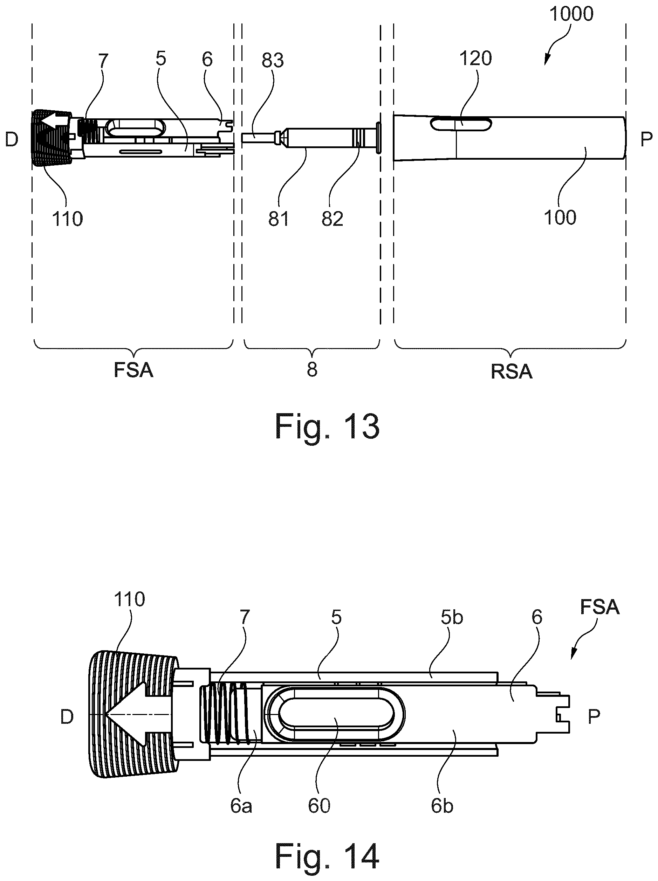

Figure 13 shows the auto-injector 1000 of the previous figures in an exploded view. The auto-injector 1000 comprises a release subassembly FSA or front subassembly FSA, respectively, a drive subassembly RSA or rear subassembly RSA, respectively, and the syringe 8. For assembling the auto-injector 1000, the syringe 8 is inserted into the front subassembly FSA or the rear subassembly RSA and afterwards the front subassembly FSA is inserted into the rear subassembly RSA. Assembling of the auto-injector 1000 will be explained in more detail further below.

Figure 14 shows the front subassembly FSA in a more detailed side view. The syringe holder 6 comprises two elongated arms 6b which extend axially and which are spaced from one another along the angular direction. The needle shroud 5 also comprises two elongated arms 5b which extend axially and which are spaced from one another along the angular direction. The needle shroud 5 and the syringe holder 6 are inserted into each other such that the arms 5b of the needle shroud 5 are located between the arms 6b of the syringe holder 6 along the angular direction. Furthermore, it can be seen that the arms 6b of the syringe holder 6 project beyond the arms 5b of the needle shroud 5 in proximal direction P.

The distal end of the syringe holder 6 is formed by a distal portion 6a in the form of a cylindrically-shaped portion 6a. This portion 6a is configured to hold the shroud spring 7. The cylindrically-shaped portion 6a is inserted into the shroud spring 7 so that an edge of the syringe holder 6 abuts against the proximal end of the shroud spring 7. The shroud spring 7 circumferentially surrounds the cylindrically-shaped portion 6a of the syringe holder 6. The shroud spring 7 may be fixed to the cylindrically-shaped portion 6a, e.g. by a glue or a mechanical radial interference with the proximal coil of the shroud spring 7.

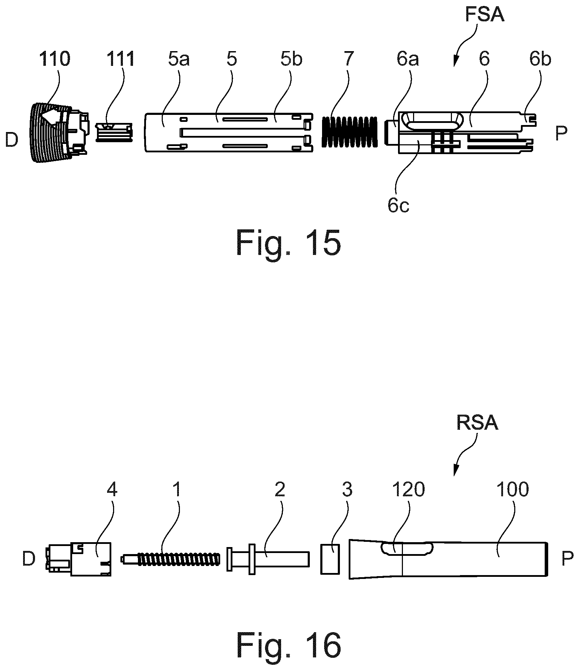

Figure 15 shows the front subassembly FSA in an exploded view. It comprises the cap 110, the grabber 111, the needle shroud 5, the shroud spring 7 and the syringe holder 6. The needle shroud 5 also comprises a distal portion 5a in the form of a cylindrically-shaped portion 5a forming the distal end of the needle shroud 5. The cylindrically-shaped portion 5a is configured to hold the shroud spring 7. This cylindrically-shaped portion 5a is shaped as a hollow cylinder so that the shroud spring 7 can be inserted into this portion 5a an so that the distal end of the shroud spring 7 abuts against a bottom area of the cylindrically-shaped portion 5a. The shroud spring 7 may be fixed to the cylindrically-shaped portion 5a, e.g. by a glue or a mechanical radial interference with the distal coil of the shroud spring 7. In this way, the needle shroud 5, the shroud spring 7 and the syringe holder 6 are coupled such that a movement of the needle shroud 5 in proximal direction P with respect to the syringe holder 6 results in a compression of the shroud spring 7. The shroud spring 7 could also be held in place by a coupling/snap between the needle shroud 5 and the syringe holder 6 at the most extended positions, e.g. by the features 54 and 61 explained further below.

As can further be seen in figure 15, the syringe holder 6 comprises a support portion 6c, which is located proximally with respect to the cylindrically shaped portion 6a and which is located between the arms 6b and the cylindrically shaped portion 6a. After inserting the syringe holder 6 into the needle shroud 5, the arms 5b of the needle shroud 5 cover the support portion 6c, i.e. are located radially outwardly with respect to the support portion 6c.

Figure 16 shows the rear subassembly RSA in an exploded view. The rear subassembly RSA comprises the housing 100, the torsion drive spring 3, the rotating collar 2, the plunger rod 1 and the drive spring holder 4. The drive spring holder 4, the rotating collar 2 and the housing 100 each have the form of a sleeve. When assembling the rear subassembly RSA, the plunger rod 1 is inserted into the rotating collar 2, the rotating collar 2 is inserted into the torsion drive spring 3 and is fixed to the torsion drive spring 3 at one connection point. The torsion drive spring 3 is inserted into the drive spring holder 4 and is connected to the drive spring holder 4 at a further connection point. The drive spring holder 4 is inserted into the housing 100.

### 2. Second exemplary embodiment of a drug delivery device

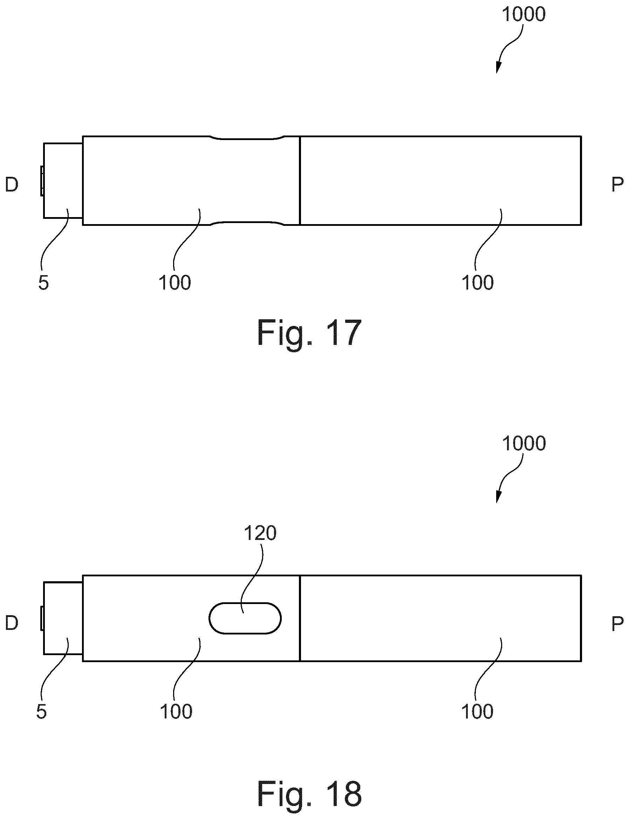

Figures 17 and 18 show a second exemplary embodiment of a drug delivery device 1000 which is again an auto-injector 1000. Like figures 1 and 2, figure 17 and 18 show the auto-injector 1000 in two different views rotated with respect to each other by 90° around the longitudinal axis A.

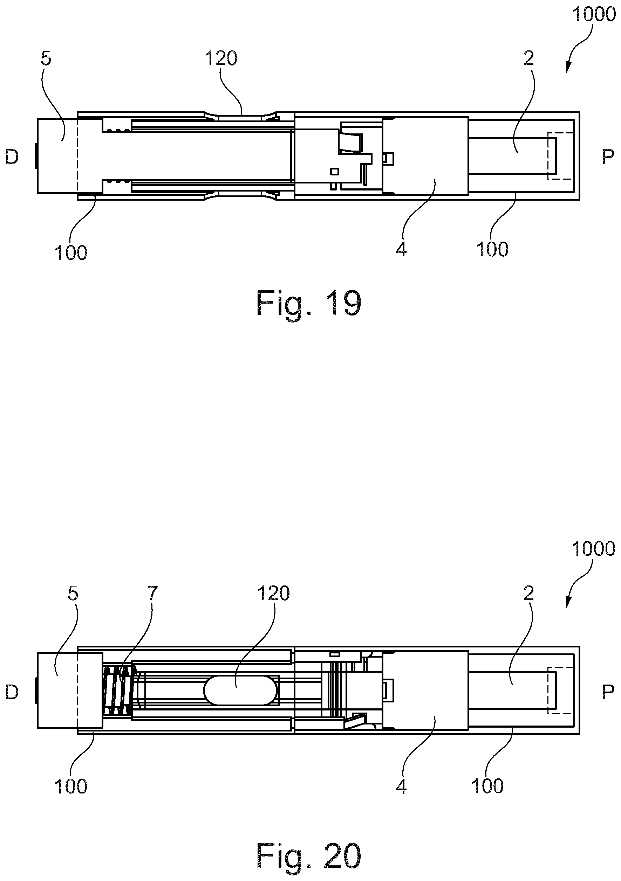

Figures 19 and 20 show the auto-injector 1000 of figures 17 and 18 in the same rotated views but with a semi-transparent housing 100.

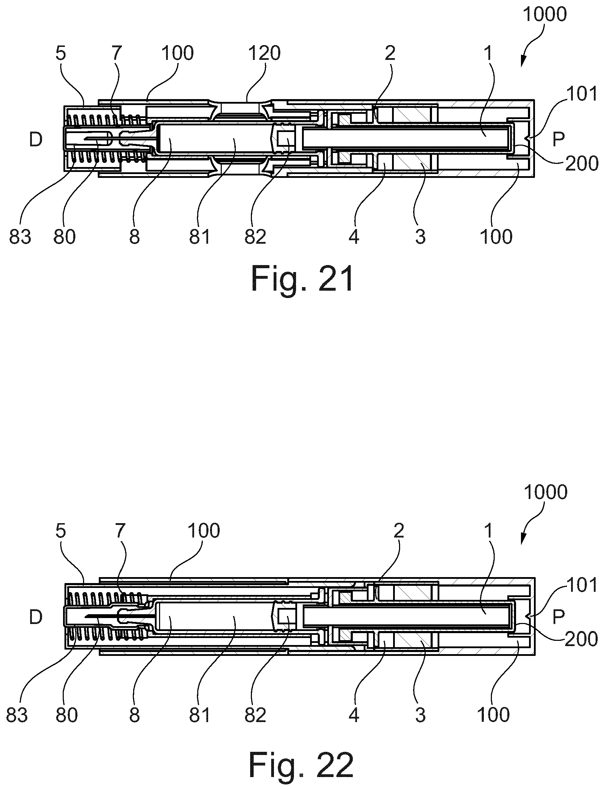

Figures 21 and 22 show the auto-injector 1000 of figures 17 and 18 in the same rotated views but now in cross-sectional view with the crossing plane comprising the longitudinal axis.

One difference between the auto-injector 1000 according to the second exemplary embodiment and the auto-injector according to the first exemplary embodiment is that, in the second exemplary embodiment, the housing 100 now comprises two parts instead of one part. A first part forming the distal part of the housing 100 and a second part forming the proximal part of the housing 100. The two parts of the housing 100 are connected to each other, e.g. with help of clips (not shown). For example, the two parts of the housing 100 are fixed to each other such that they are neither axially nor rotationally nor radially movable with respect to each other.

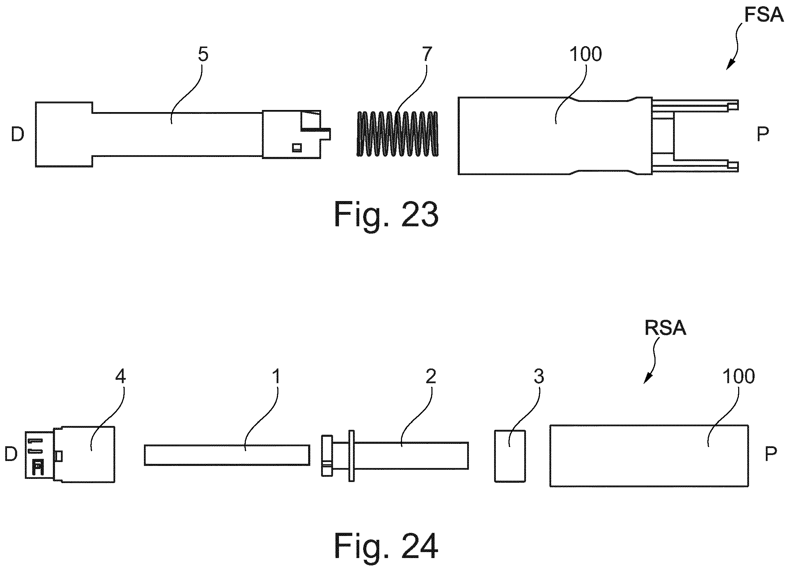

Figure 23 shows a front subassembly FSA of the auto-injector 1000 according to the second exemplary embodiment in an exploded view. The first part of the housing 100 is assigned to the front subassembly FSA. The needle shroud 5 may be inserted into this first part of the housing 100. The shroud spring 7 is connected to the needle shroud 5 and the first part of the housing 100 so that a movement of the needle shroud 5 in proximal direction with respect to the first part of the housing 100 results in a compression of the shroud spring 7. In difference to the first exemplary embodiment, the auto-injector according to the second exemplary embodiment does not comprise a syringe holder with two arms spaced in angular direction. Instead of such a syringe holder, the first part of the housing 100 is configured to hold a medicament container, e.g. in an axially and/or rotationally fixed manner. The first part of the housing 100 circumferentially completely surrounds the needle shroud 5.

An exploded view of the rear subassembly RSA of the auto-injector 1000 according to the second exemplary embodiment is shown in figure 24. This rear subassembly is basically identical to the rear subassembly RSA of the first exemplary embodiment. Only the second part of the housing 100 assigned to the rear subassembly RSA may be shorter than the housing 100 of the first exemplary embodiment.

### 3. Drive Mechanism

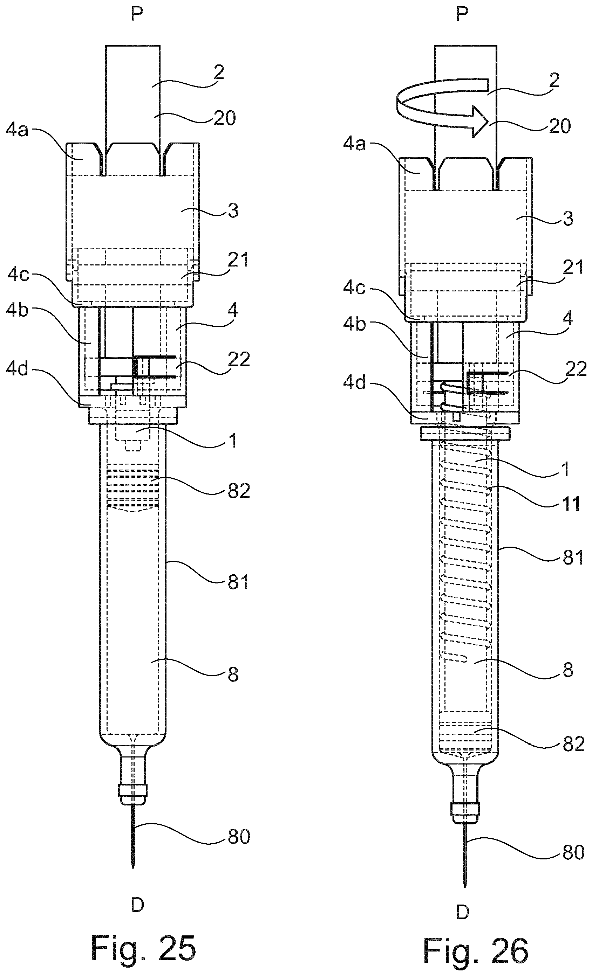

The conversion of the rotational movement of the rotating collar 2, induced by the torsion drive spring 3, into an axial movement of the plunger rod 1 (drive mechanism) is explained in more detail in the following in connection with figures 25 to 27.

Figures 25 and 26 show a part or arrangement of the auto-injector 1000 of the first and second exemplary embodiment in different positions during usage. The shown part comprises the rear subassembly (only the housing is not shown) and a syringe 8. In figure 25, the auto-injector 1000 is in the first locked state and in figure 26 the auto-injector is in the released state.

As can be seen in figures 25 and 26, the drive spring holder 4 comprises two hollow sections 4a, 4b, which may both be hollow cylindrically-shaped. The two sections 4a, 4b are arranged behind each other along the longitudinal axis. The first section 4a is located more proximally and has a greater inner diameter and a greater outer diameter than the second section 4b.

The rotating collar 2 is received in the drive spring holder 4. A proximal end of the rotating collar 2 projects out of the drive spring holder 4 in proximal direction P. The rotating collar 2 comprises a shaft 20, and two portions 21, 22 with larger diameters than the shaft 20. The two portions 21, 22 are axially spaced from one another and are connected via the shaft 20. In this exemplary embodiment, the two portions 21, 22 are disc-shaped but other shapes might also be possible. The first portion 21 has a greater diameter than the second portion 22. The first portion 21 is located in the first section 4a of the drive spring holder 4 and the second portion 22 is located in the second section 4b of the drive spring holder 4. The diameters of the portions 21, 22 are substantially the same as the inner diameters of the assigned sections 4a, 4b but sufficiently smaller to allow a rotation of the rotating collar 2 with respect to the drive spring holder 4. Moreover, the diameter of the first portion 21 is greater than the inner diameter of the second section 4b which limits the axial movement of the rotating collar 2 in distal direction D.

As can be further seen in figure 25, in the first locked state, the second portion 22 is offset in proximal direction P from a second bottom ring 4d of the drive spring holder 4. Likewise, the first portion 21 is offset in proximal direction P from a first bottom ring 4c of the drive spring holder 4.

The torsion drive spring 3 is received in the first section 4a and is fixed to the first section 4a at a connection point. The rotating collar 2 is received in the torsion drive spring 3 so that the torsion drive spring 3 circumferentially surrounds the shaft 20 of the rotating collar 2 at a proximal side of the first section 21. The shaft 20 of the rotating collar 2 is connected to the torsion drive spring 3 at a further connection point. The first portion 21 is offset with respect to the torsion drive spring 3 in distal direction D. In the first locked state, shown in figure 25, the torsion drive spring 3 is biased and induces a torque onto the rotating collar 2. The rotating collar 2 is prevented from a rotation with help of the first locking mechanism explained further below.

The plunger rod 2 is received in the rotating collar 2. In the first locked state, a portion of the plunger rod 1 projects from the rotating collar 2 in distal direction D. The stopper 82 of the syringe 8 is offset from the distal end of the plunger rod 1 in distal direction D.

Figure 26 shows the part or arrangement of the auto-injector in the released state. The first locking mechanism has been released so that the rotating collar 2 was no longer prevented from rotating. Due to the torque induced by the drive spring 3, the rotating collar 2 rotates in a first rotational direction (clockwise or anti-clockwise) inside the drive spring holder 4. The rotating collar 2 and the plunger rod 1 are operatively coupled via a threaded interface. In the present case, the plunger rod 1 comprises an external thread 11 and the rotating collar 2 comprises an internal thread (not visible) engaging with the external thread 11 of the plunger rod 1. The coupling via the threaded interface is such that the rotation of the rotating collar 2 in the first rotational direction is converted into a movement of the plunger rod 1 in distal direction D.

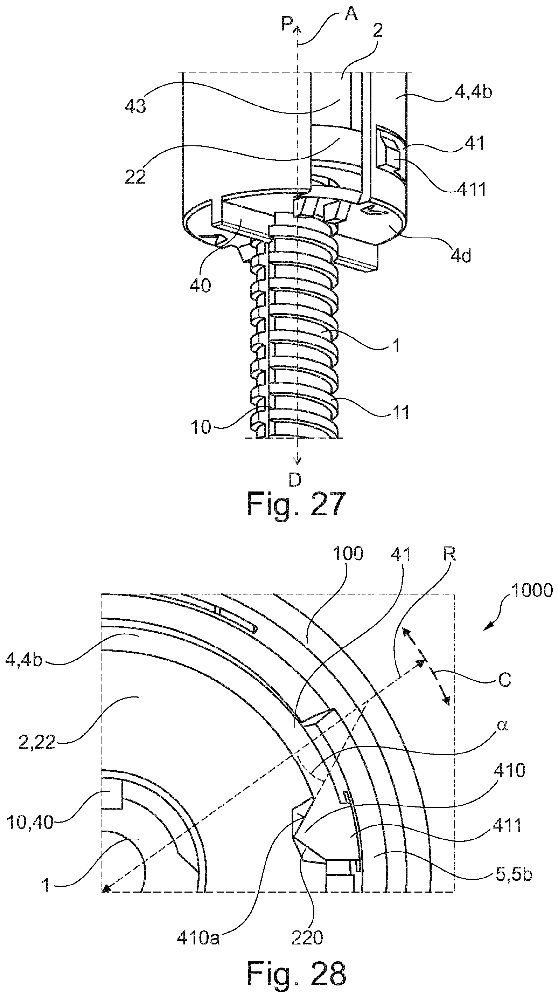

During the axial movement of the plunger rod 1 induced by the rotation of the rotating collar 2, the plunger rod 1 itself does not rotate. This is realized by a coupling between the plunger rod 1 and the drive spring holder 4 via a splined interface. This is further illustrated in figure 27 showing a three-dimensional view of the part/arrangement of the auto-injector. The splined interface is realized by protrusions 40 of the drive spring holder 4 projecting in distal direction D from the second bottom ring 4d and engaging with or projecting into grooves 10 of the plunger rod 1, respectively. The grooves 10 extend along the longitudinal axis A, i.e. run essentially parallel to the longitudinal axis A. The grooves 10 are arranged opposite each other on the plunger rod 1. Instead of two grooves, as shown in figure 27, one groove and one corresponding protrusions 40 may be sufficient. However, more than two grooves 10 and associated protrusions 40 may also be used.

In the exemplary embodiments, the splined interface is in close proximity to the threaded interface, e.g. with a distance of at most 1 cm or at most 0.5 cm. This is beneficial since the torque on the plunger rod 1 is resolved over a short distance reducing the stresses within the plunger rod 1. The plunger rod 1 is often a small member likely to deform.

As can be further seen in figure 26, the rotating collar 2 does not only rotate but also moves axially in the proximal direction P, as already mentioned before. The movement in proximal direction P preferably starts immediately when the rotation is started. In this way the needle shroud 5 may reextend upon premature removal from the skin. The break loose force of the stopper 82 is typically 5 N or more. The ability of the torsion drive spring 3 to resolve axial loads may be smaller than this.

In the released state, the plunger rod 1 pushes the stopper 82 in distal direction D until the stopper 82 hits against a bottom region of the cartridge 81. A further distal movement of the stopper 82 and the plunger rod 1 is then prevented. After movement of the plunger rod 1 and the rotating collar 2 is finished, a portion of the plunger rod 1 is still received in the rotating collar 2.

An example of the dimensions of the plunger rod 1 is as follows: The plunger rod 1 has a diameter of 8.0 mm and the pitch of the outer thread is 3.17 mm. The coefficient of friction is 0.3. The mean contact radius, i.e. the position of the threaded face from the central axis of the plunger rod 1, is 3.75 mm.

An example of the torsion drive spring is 3 as follows: The material is polished and blued SAE 1095 steel. The height of the torsion drive spring is 12.0 mm, the thickness of the material is 0.168 mm, the length is 840.749 mm, the outer diameter is 20.0 mm, the arbor diameter is 10.0 mm. The bending stress limit is 2000 N·mm⁻², the Youngs Modulus is 20000 N·mm⁻², the number of revolutions before being biased is 3.

In general, the following conditions for the torsion drive spring turned out to be advantageous: The arbor diameter is between 12 to 25 times the thicknesses of the material. The length is between 5000 to 15000 times the thickness. The area of the torsion drive spring 3 is half the area of the drive spring holder 4 (e.g. in the first section 4a) +- 10%. The bending stress for tempered polished and blued SAE 1095 steel should not exceed 2000 MPa.

An example of the used syringe 8 might be as follows: The drug inside the cartridge 81 has a volume of 2 ml. The viscosity of the material is 50 cP at room temperature. The inner needle diameter is 0.29 mm. The inner cartridge diameter is 8.65 mm. The friction of the stopper 82 is 10 N. The stopper gap, i.e. the initial clearance between the proximal end of the stopper 82 and the distal end of the plunger rod 1, is 2 mm.

### 4. First locking mechanism and release of the first locking mechanism

The previously mentioned first locking mechanism or first rotation-locking mechanism, respectively, and how it is released is described in further detail in the following in connection with the figures 28 to 33.

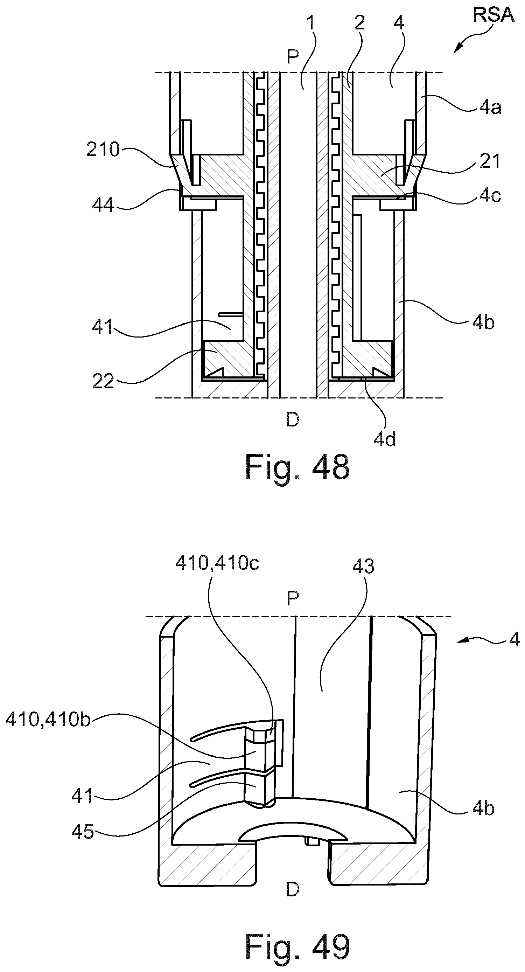

Figure 28 illustrates a cross-sectional view of the auto-injector 1000 of the first and second exemplary embodiment with the cutting plane being perpendicular to the longitudinal axis A and running through the second portion 22 of the rotating collar 2. As can be seen, the drive spring holder 4 comprises a displaceable element 41 in the form of a resilient arm (see also figures 27 and 49). The resilient arm 41 is integrally formed with the drive spring holder 4 and is arranged in the second section 4b of the drive spring holder 4. The resilient arm 41 is oriented circumferentially, i.e. a main extension direction of the resilient arm 41 is along the angular direction C. One end of the resilient arm 41 is connected to the drive spring holder 4, the other end is free and movable in radial direction R.

The resilient arm 41 comprises a protrusion 410 projecting radially inwardly, i.e. in a radial direction pointing towards the longitudinal axis A. The protrusion 410 tapers radially inwardly. The protrusion 410 comprises a beveled surface 410a, which essentially runs parallel to the longitudinal axis A and which is tilted with respect to the radial direction R and with respect to the angular direction C. For example, the angle α between the beveled surface 410a and the radial direction R is at least 10° and at most 80°, preferably between 30° and 55°.

In the first locked state, shown in figure 28, the resilient arm 41 is in a first radial position in which the protrusion 410 engages or projects into a recess 220 of the second portion 22 of the rotating collar 2, respectively. In this way a rotation-lock interface is formed, coupling the resilient arm 41 and the rotating collar 2 and preventing the rotating collar 2 from a rotation. The first radial position may be the relaxed position of the resilient arm 41 which it would occupy if no further forces pointing radially inwardly and radially outwardly were acting on the resilient arm 41. Alternatively, the resilient arm 41 may be biased in the first radial position, such that the first radial position is a stressed position of the resilient arm 41.

As long as the resilient arm 41 is in the first radial position in which the protrusion 410 projects into the recess 220, a rotation of the rotating collar 2 in the first rotational direction induced by the torsion drive spring 3 is prevented. However, the torque acting on the rotating collar 2 presses a surface of the second portion 22 delimiting the recess 220 against the beveled surface 410a of the protrusion 410 of the resilient arm 41. This results in a force trying to move the resilient arm 41 radially outwardly from the first radial position into a second radial position. In other words, the torque induced by the torsion drive spring 3 biases the resilient arm 41 radially outwardly. If a movement in radial outward direction would be allowed, the first locking mechanism would be released automatically and the auto-injector 1000 would transfer into the released state.

In the first locked state, an arm 5b of the needle shroud 5 is located at the height of, i.e. axially overlapping or aligned with, the resilient arm 41 and prevents the resilient arm 41 from moving radially outwardly out of and away from the first radial position. Indeed, the resilient arm 41 abuts against the needle shroud 5 in outward radial direction such that an outward radial movement is blocked. The resilient arm 41 comprises a further protrusion 411 which projects radially outwardly and which abuts against the needle shroud 5. An outward radial movement of the needle shroud 5 is prevented, e.g. by the housing 100 circumferentially surrounding the needle shroud 5.

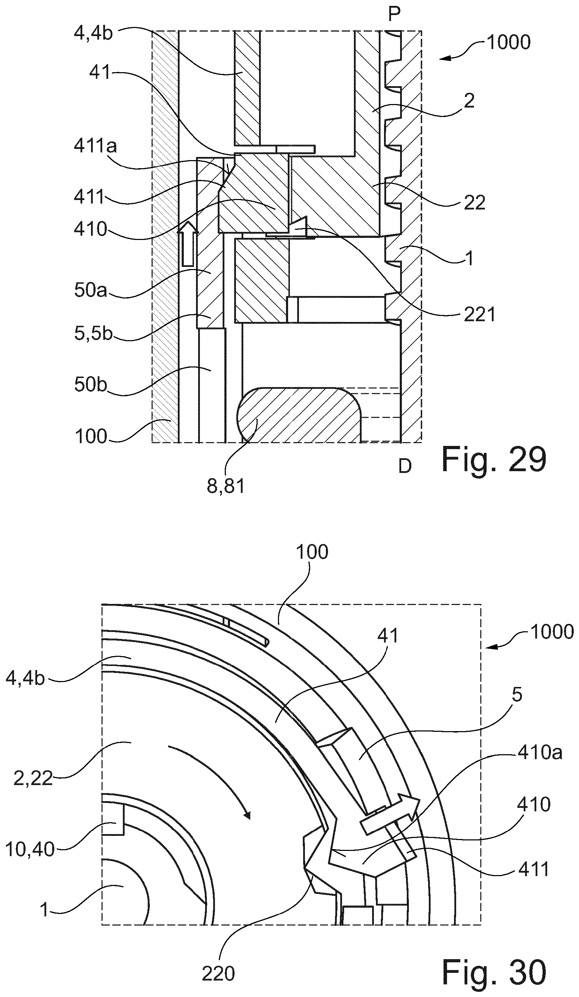

Figure 29 shows a section of the auto-injector 1000 in the same state as in figure 28 but now in a cross-sectional view with the longitudinal axis lying in the cutting plane. There it can be seen that the arm 5b of the needle shroud 5 in fact comprises a first section 50a, namely a wall portion, and a second section 50b, namely a recess, e.g. a cut-out. The recess 50b is offset in distal direction D with respect to the wall portion 50a. In the first locked state, the needle shroud 5 is in its extended position, in which the wall portion 50a blocks the outward radial movement of the resilient arm 41.

Figure 29 further indicates that the needle shroud 5 can be moved from its extended position into a retracted position which would result in an overlap or alignment between the recess 50b and the resilient arm 41 in axial direction and rotational direction. Movement of the needle shroud 5 in proximal direction P requires a force, also called activation force, which includes the force needed to compress the shroud spring 7 and the friction force resulting from the resilient arm 41 being pressed against the needle shroud 5.

As an numerical example: Assuming a torque induced by the torsion drive spring 3 onto the rotating collar 2 of 102 Nmm, a radius at which the rotating collar 2 abuts against the protrusion 410 of 7.5 mm and an angle α of 39° would result in a force on the resilient arm 41 in radial direction of about 10.57 N. Assuming a friction coefficient of 0.3, the friction force would be about 3.17 N. Assuming further that the force for compressing the shroud spring 7 is about 6 N, the activation force would be about 9 N.

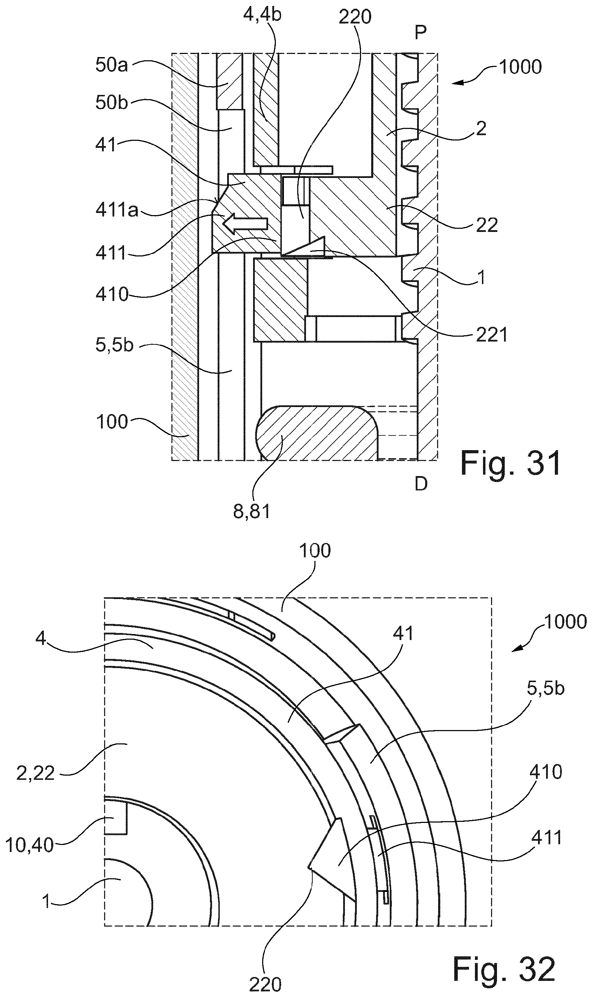

Figures 30 and 31 show sections of the auto-injector 1000 which corresponds to the sections shown in figures 28 and 29. Now the needle shroud 5 has been moved in its retracted position (by overcoming the activation force). This movement releases the first locking mechanism so that the auto-injector 1000 is switched from the first locked state into the released state. As the recess 50b of the needle shroud 5 is now at the height of the resilient arm 41, the outward radial movement of the resilient arm 41 is no longer blocked. The resilient arm 41 automatically - induced by the torque on the rotating collar 2 - leaves its first radial position and deflects into a second radial position in which the protrusion 410 no longer projects into the recess 220, thus the rotation-lock interface is resolved and the first locking mechanism is released. As a result of this, the rotation of the rotating collar 2 is no longer prevented. The rotating collar 2 starts to rotate (see figure 30) due to the force induced by the drive spring 3, thereby forcing the plunger rod 1 into an axial movement.

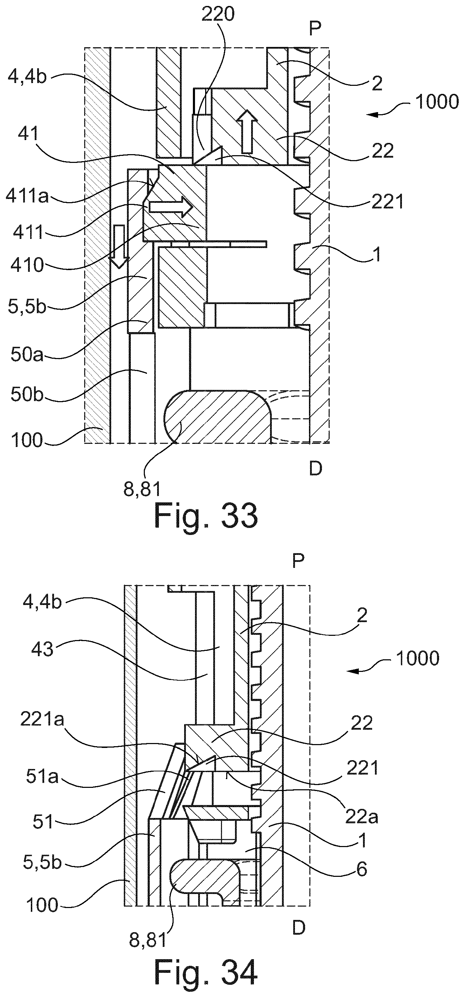

Figure 32 and 33 show sections of the auto-injector 1000 which corresponds to the sections shown in figures 28 and 29. Now, the auto-injector 1000 is switched into a third locked state or post-released state. For example, the distal end of the auto-injector 1000 has been removed from the body so that the needle shroud 5 automatically moves from the retracted position back into the extended position induced by the shroud spring 7.

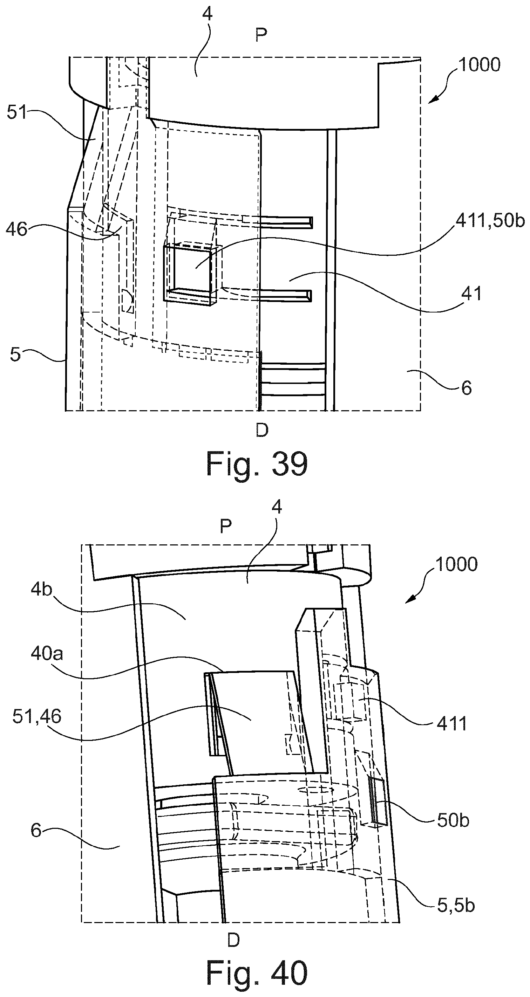

The protrusion 411 of the resilient arm 41 comprises a slide feature 411a in the form of a beveled surface 411a. The beveled surface 411a and the longitudinal axis may include, e.g., an angle between 10° and 80° inclusive. An edge of the needle shroud 5 delimiting the recess 50b in proximal direction P may contact this beveled surface 411a when the needle shroud 5 moves in distal direction D. Due to the beveled surface 411a, the resilient arm 41 is pushed radially inwardly when the edge hits the protrusion 411. In this way, the movement of the needle shroud 5 back into the retracted position is possible without the needle shroud 5 jamming up with the resilient arm 41. The slide feature may additionally or alternatively be formed in the needle shroud 5 (see figures 39 and 40).

In the case the resilient arm 41 indeed abuts against the edge of needle shroud 5 when the needle shroud 5 is moved in distal direction D, the movement of the resilient arm 41 in inward radial direction is possible, since the rotating collar 2, particularly the second portion 22 of the rotating collar 2, has moved in proximal direction P. Thus, the second portion 22 is now offset in proximal direction P with respect to the resilient arm 41. For this reason, it is particularly beneficial if the rotating collar 2 moves in the proximal direction immediately when the plunger rod 1 starts to move in distal direction, i.e. before the plunger rod 1 hits the stopper 82. If the user lifts the auto-injector 1000 early from the skin, e.g. before the drug is started to be administered, the needle shroud 5 can then still move back in distal direction and the third locking mechanism explained below can be activated.

### 5. Third locking mechanism / post-released locking mechanism

A third locking mechanism or post-released locking mechanism, respectively, is described in further detail in the following in connection with the figures 34 to 40.

Figures 34 to 38 illustrate a first exemplary embodiment of the third locking mechanism. This mechanism is configured to prevent the needle shroud 5 from being moved from the extended position into the retracted position after the drug has been delivered or after the autoinjector has once been activated. Thus, the risk of injuries due to an exposed needle may be reduced. This third locking mechanism may be used in all exemplary embodiments of the auto-injector 1000 described herein.

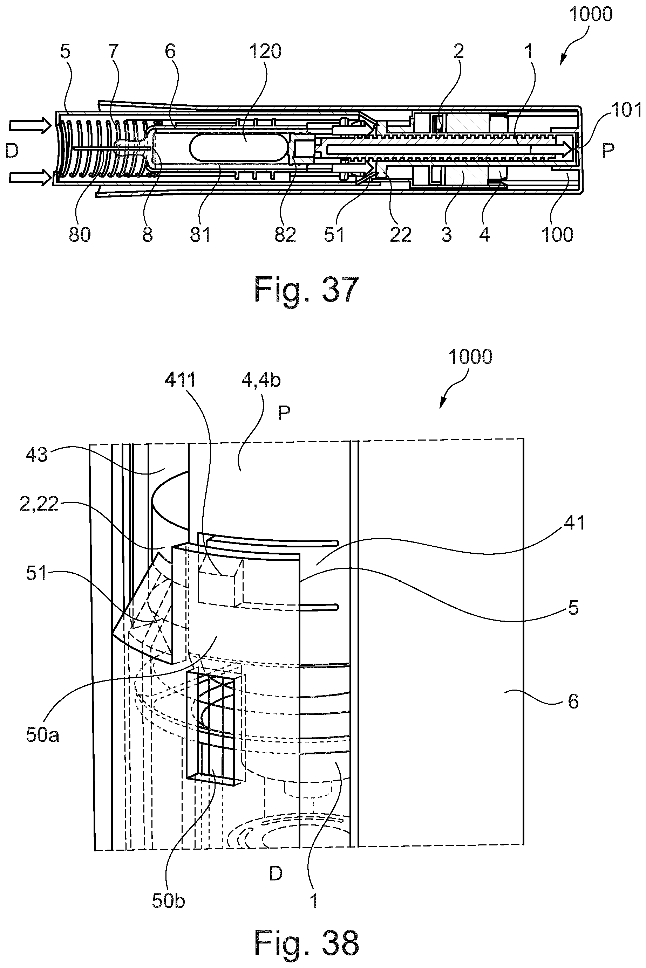

Figure 34 shows again a cross-sectional view of a section of the auto-injector 1000 with the cutting plane comprising the longitudinal axis A. However, the cutting plane is rotated compared to what is shown in, e.g. figure 33 (see figure 38 for a perspective view). It can be seen in figure 34 that the arm 5b of the needle shroud 5 comprises a first stop feature 51 in the form of a displaceable element 51 which is located at the proximal end of the arm 5b. The displaceable element 51 is a resilient arm 51 which is integrally formed with the rest of the needle shroud 5 and, therefore, is axially and rotationally fixed to the rest of the needle shroud 5. Thus, the resilient arm 51 moves in axial direction when the needle shroud 5 is moved in axial direction.

As can be seen in figure 38, the resilient arm 51 is located on the same height as the wall portion 50a when seen along to the longitudinal axis A and is arranged offset from the wall portion 50a in the angular direction C.

Simultaneously to extending in proximal direction P, the resilient arm 51 also extends radially inwardly, i.e. a main extension direction of the resilient arm 51 has a component along the proximal direction P and a component along the inward radial direction. Thus, a proximal end of the resilient arm 51 is located further radially inwardly than a distal end of the resilient arm 51. The proximal end of the resilient arm 51 is free and displaceable in the radial direction. The distal end of the resilient arm 51 is connected to the rest of the needle shroud 5. A kink is formed between the distal end of the resilient arm 51 and the rest of the needle shroud 5.

In figure 34, the auto-injector 1000 is in the first locked state (also referred to as initial state or pre-released state), in which a rotation of the rotating collar 2 is blocked by the first locking mechanism as described before. The resilient arm 51 is in a first radial position, which may be a biased position of the resilient 51. The resilient arm 51 is held in the first radial position and is prevented from moving radially inwardly by the second portion 22 of the rotating collar 2. In the present case, the drive spring holder 4 comprises a recess 43, namely a cut-out 43, into which the resilient arm 51 projects. The resilient arm 51 abuts against the second portion 22 in inward radial direction.

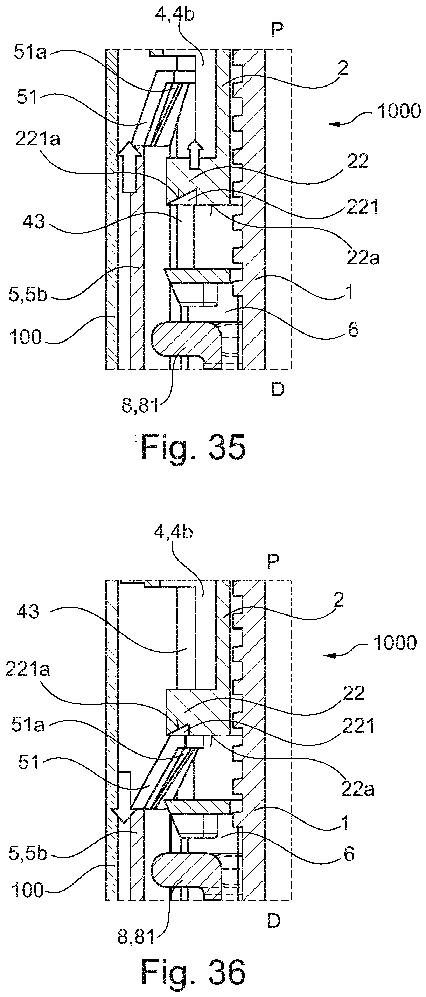

Figure 35 shows a section of the auto-injector 1000 in a position during usage, when the needle shroud 5 is moved from its extended position into the retracted position so that the auto-injector 1000 switches into the released state. Together with the needle shroud 5, the resilient arm 51 has moved in proximal direction P such far that the second portion 22 of the rotating collar 2 does no longer hold the resilient arm 51 in the first radial position. This allowed the resilient arm 51 to move radially inwardly into a second radial position. In the released state of the auto-injector 1000 and the needle shroud 5 being in the retracted position, the resilient arm 51 is offset with respect to the second portion 22 in proximal direction P.

In the released state of the auto-injector 1000, the rotating collar 2 moves in proximal direction P from a nonlocking position into a locking position, as it is indicated in figure 35.

Figure 36 shows a section of the auto-injector 1000 in a third locked state, also referred to as post-released state, which is a state after usage, i.e. after the drug has been dispensed. The third locked state is a state after the released state. In this third locked state, the needed shroud 5 is again in its extended position. As can be seen in figure 36, the second portion 22 has moved in proximal direction P such far that the resilient arm 51 is now offset in distal direction D with respect to the second portion 22 so that the second portion 22 can no longer hold the resilient arm 51 in the first radial position. Therefore, in the third locked state, the resilient arm 51 is in the second radial position. When trying to move the needle shroud 5 from the extended position towards the retracted position, the resilient arm 51 in the second radial position hits against a second stop feature 22a, namely a surface of the second portion 22 which runs essentially perpendicularly to the longitudinal axis and faces in distal direction D. This prevents a further movement of the needle shroud 5 in proximal direction P. For example, the auto-injector 1000 is configured such that, in the third locked state, the resilient arm 51 hits against the surface 22a of the second portion 22 when moving the needle shroud 5 in proximal direction P before the needle is exposed.

When the resilient arm 51 hits against the surface 22a of the second portion 22, a lock interface is formed between the resilient arm 51 and the surface 22a. For this purpose, a recess 221 or notch 221 is formed in the surface 22a which engages with the proximal end of the resilient arm 51 when the resilient arm 51 hits against the surface 22a. The recess 221 is delimited by a beveled surface 221a which is tilted with respect to the longitudinal axis A and the radial direction. For example, an angle between the beveled surface 221a and the longitudinal axis and/or the radial direction is between 10° and 80° inclusive. When the proximal end of the resilient arm 51 engages into the recess 221, the resilient arm 51 hits against the beveled surface 221a and slides along the beveled surface 221a thereby being forced to move radially inwardly. The recess 221 with the beveled surface 221a thus prevents the resilient arm 51 from sliding along the surface 22a in outward radial direction.

The surface 22a of the second portion 22 may circumferentially extend around the longitudinal axis and/or the rotational axis of the rotating collar 2 by at least 270° and may have a constant geometrical form along its extension along the angular direction. In this way, the functionality of the third locking mechanism is almost independent on how far the rotating collar 2 has rotated in the released state.

As can be further seen in figures 34 to 36, the resilient arm 51 comprises a slide feature 51a in the form of a ramp 51a. During movement of the needle shroud 5 from the retracted position into the extended position, the ramp 51 hits against a proximal edge of the second portion 22. The ramp 51a is designed such that it forces the resilient arm 51 to slide along the edge of the second portion 22 so that the resilient arm 51 is pushed radially outwardly. This allows the resilient arm 51 to pass the second portion 22 without being jammed up with the second portion 22. After having passed the second portion 22 during movement towards the extended position, the resilient arm 51 springs back into the second radial position.

Figure 37 shows the auto-injector 1000 in a cross-sectional view in the third locked state. As can be seen, the needle shroud 5 cannot be moved such far in proximal direction P that the needle 80 is exposed because the resilient arm 51 hits against the surface 22a of the second portion 22 before.

Figures 39 and 40 illustrate a second exemplary embodiment of the third locking mechanism. Also this exemplary embodiment of the third locking mechanism may be used in all exemplary embodiments of the auto-injector described herein.

The main difference to the first exemplary embodiment is that, in the third locked state of the auto-injector 1000, when moving the needle shroud 5 towards the retracted position, the resilient arm 51 does not hit against a stop feature axially fixed to the rotating collar 2 but against a stop feature 40a axially fixed to the drive spring holder 4. The stop feature 40a is formed by an edge of the drive spring holder 4. The edge 40a delimits a recess/cut-out in the drive spring holder 4 in proximal direction P.

A flap 46, which is axially fixed to the drive spring holder 4, e.g. integrally formed with the drive spring holder 4, partially fills this recess. A distal end of the flap 46 is connected to the drive spring holder 4 and a proximal end of the flap 46 is free and displaceable in radial direction. The proximal end of the flap 46 is spaced from the edge 40a by a small gap.

In the first locked state, when the needle shroud 5 is still in the extended position, the rotating collar 2, particularly the second portion 22 of the rotating collar 2, abuts against the flap 46 of the drive spring holder 4 in outward radial direction and holds the flap 46 in a first radial position, in which the flap 46 substantially terminates flush with the edge 40a in outward radial direction. The second portion 22 prevents the flap 46 from being displaced in the inward radial direction. On the other hand, the flap 46 abuts against the resilient arm 51 of the needle shroud 5. In the first radial position of the flap 46, the flap 46 holds the resilient arm 51 in its first radial position.

When now moving the needle shroud 5 in proximal direction P, the resilient arm 51 can pass the edge 40a without jamming up with the edge 40a, since the flap 46 terminates flush with the edge 40a and since the flap 46 is held in its first radial position by the second portion 22. Moving the needle shroud 5 further into its retracted position releases the first locking mechanism, the auto-injector 1000 switches from the first locked state into the released state and the rotating collar 2 together with the second portion 22 moves in proximal direction P into a locking position. The needle shroud 5 being in its retracted position is shown in figure 39.

When moving the needle shroud 5 back from its retracted position into the extended position, the resilient arm 51 passes the edge 40a and stops at the height of the flap 46. This position is shown in figure 40. The auto-injector 1000 is now in the third locked state. The resilient arm 51 and optionally also the flap 46 may be biased in inward radial direction. Thus, the resilient arm 51 and the flap 46 move radially inwardly and each reach a second radial position. This is possible because the elements are no longer held by the second portion 22 of the rotating collar 2 in their respective first radial position.

The flap 46 being in the second radial position does no longer terminate flush with the edge 40a of the drive spring holder 4. Thus, when moving the needle shroud 5 from the extended position towards the retracted position, the resilient arm 51 will hit against the edge 40a which prevents a further movement of the needle shroud 5 in proximal direction P.

### 6. Drop protection mechanism

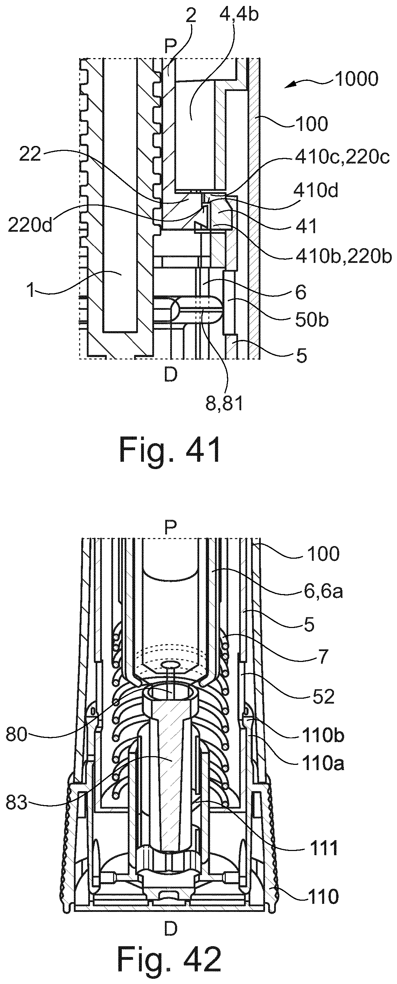

An exemplary embodiment of a drop protection mechanism is described in further detail in the following in connection with the figures 41 and 42. The drop protection mechanism shall prevent the release of the first locking mechanism when the auto-injector 1000 is unintentionally dropped. In fact, when the auto-injector 1000 of the exemplary embodiments described herein is in the first locked state, a movement of the rotating collar 2 in proximal direction P would result in a release of the first locking mechanism.

Figure 41 shows a section of the auto-injector 1000 of the first and second exemplary embodiments in cross-sectional view illustrating a first part of the drop protection mechanism. In the first locked state of the auto-injector 1000 and when the needle shroud 5 is still in the extended position (initial position), the second portion 22 and the resilient arm 41 engage with each other (protrusion 410 projects into recess 220) and this engagement is retained by the needle shroud 5 holding the resilient arm 41 in its radial position as explained in connection with the first locking mechanism. The engagement, however, also establishes an axial-lock interface preventing the rotating collar 2 from an axial movement at least in proximal direction P.

For this purpose, the protrusion 410 of the resilient arm 410 is a stepped protrusion having two section 410b, 410c (see also figure 49). The recess 220 in the second portion 22 of the rotating collar 2 is a stepped recess also having two sections 220b, 220c. The sections 410b, 410c are connected by a surface 410d running essentially perpendicularly to the longitudinal axis. The sections 220b, 220c are also connected by a surface 220d running essentially perpendicularly to the longitudinal axis. The surface 220d is located more distally than the surface 410d. These surfaces 220d, 410d abut or hit against each other when the rotating collar 2 is moved in proximal direction P and, in this way, the rotating collar 2 is prevented from moving in proximal direction P as long as the protrusion 410 projects into the recess 220.

The first part of the drop protection mechanism described in connection with figure 41 could, however, be released when the needle shroud 5 would unintentionally be moved in proximal direction P. Therefore, in one exemplary embodiment, the drop protection mechanism comprises a second part illustrated in connection with figure 42.

Figure 42 shows a section of the auto-injector in a cross-sectional view with the cutting plane running parallel to the longitudinal axis A. Shown is the distal end of the auto-injector with the cap 110 still being coupled to the housing 100. The cap 110 is in its most proximal position and cannot be moved further in proximal direction P with respect to the housing 100 since it hits against the housing 100 when moved in this direction. The cap 110 comprises a radially displaceable cap-lock element 110a, namely a resilient arm 110a, with a protrusion 110b protruding radially inwardly and engaging into a cap-lock element 52, namely a recess 52, particularly a cut-out 52, in the needle shroud 5.

In figure 42, the auto-injector 1000 is shown when it is dropped which results in a proximal movement of the needle shroud 5. The needle shroud 5, particularly an edge of the needle shroud 5 delimiting the recess 52 in distal direction D, hits against the protrusion 110b due to its proximal movement. This prevents a further movement of the needle shroud 5 in proximal direction P as long as the cap 110 is coupled to the housing 100. Thus, the needle shroud 5 cannot reach the retracted position in which it would no longer hold the resilient arm 41 in its radial position.

In the position shown in Figure 42, the resilient arm 110a cannot or only slightly be moved in outward radial direction as the housing 1000 circumferentially surrounds the resilient arm 110a and abuts or almost abuts against the resilient arm 110a thereby preventing an outward radial movement of the resilient arm 110a.

The protrusion 110b is located at a proximal end of the resilient arm 110a of the cap 110. Normally, when the drug delivery device is not dropped, the edge of the needle shroud 5 delimiting the recess 52 in distal direction D is located further distal as to what is shown in figure 42. When removing the cap 110, the cap 110 is moved in distal direction D until the protrusion 110b hits against said edge of the recess 52. The resilient arm 110a can then move in radial outward direction, because in this position of the cap 110, the housing 100 does not prevent the resilient arm 110a from being moved radially outwardly. The resilient arm 110a can disengage from the recesses 52 and the cap 110 can be completely removed. The protrusion 110b has a beveled surface (slide feature) which hits against the edge of the recess 52 and thereby forces the resilient arm 110a to deflect radially outwardly when the cap 110 is moved in distal direction D.

### 7. Subassemblies, assembling and second locking mechanism

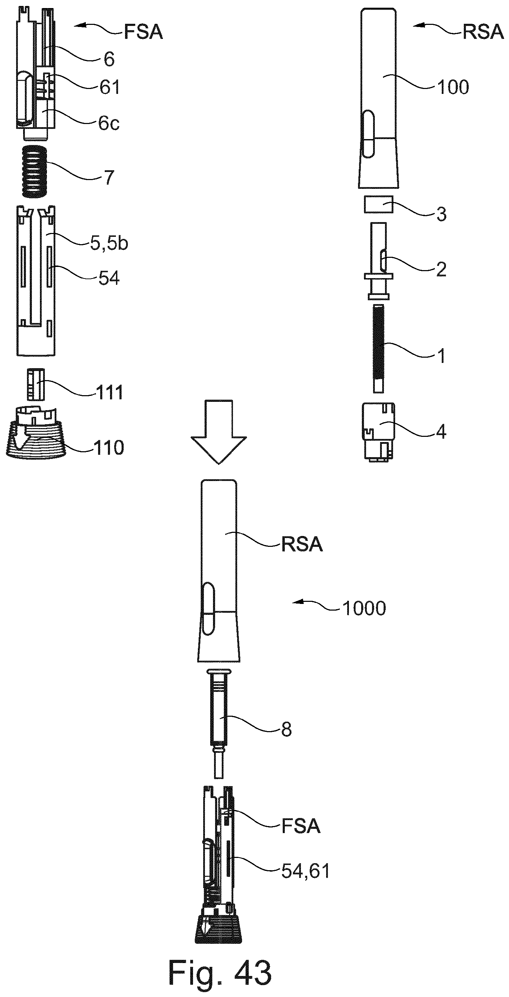

Figure 43 shows the front subassembly FSA (also referred to as release subassembly FSA or container-holder subassembly FSA) and the rear subassembly RSA (also referred to as drive subassembly RSA) of the auto-injector according to the first exemplary embodiment in an exploded view as well as a position during assembling the front subassembly FSA and the rear subassembly RSA to an auto-injector 1000. These figures correspond to the figures 13, 15 and 16. It is therefore mainly referred to the description in connection with these figures.

What can be seen in figure 43 is that the support portion 6c of the syringe holder 6 comprises a first rotation-lock features 61 in the form of protrusions 61 or ribs 61 which protrude in outward radial direction and which have a main extension direction along the longitudinal axis. These ribs 61 are configured to engage with second rotation-lock features 54 in the form of recesses 54, particularly slots 54, in the arms 5b of the needle shroud 5. The recesses 54 are also elongated with a main extension direction along the longitudinal axis and are longer than the ribs 61 so that, when engaged, a relative axial movement between the needle shroud 5 and the syringe holder 6 is possible.

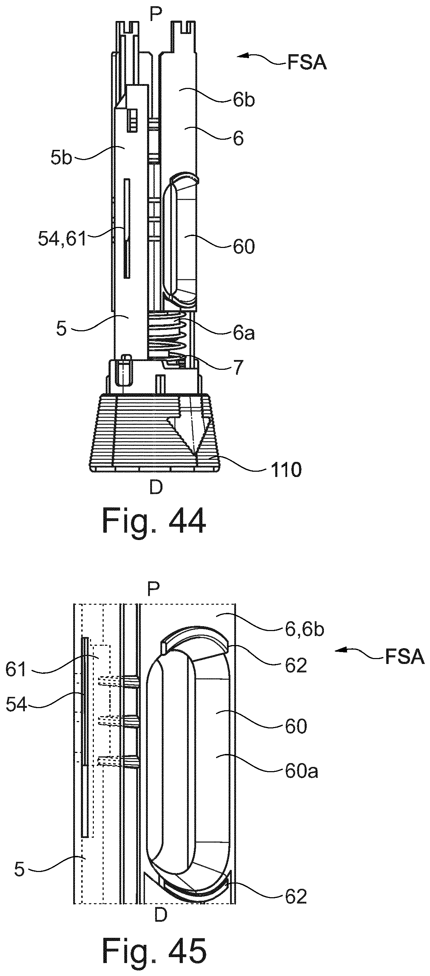

Figure 44 shows the front subassembly FSA in perspective view. As previously described, the needle shroud 5 comprises two arms 5b which are positioned, along the angular direction, between two arms 6b of the syringe holder 6. The arms 6b of the syringe holder 6 project beyond the arms 5b of the needle shroud 5 in proximal direction P. The needle shroud 5 and the syringe holder 6 are coupled by the shroud spring 7 and the rotation-lock features 61, 54 so that the needle shroud 5 can be moved axially but not rotationally with respect to the syringe holder 6.

In figure 45, a section of the front subassembly FSA of figure 44 is shown. Windows 60 are formed in the arms 6b of the syringe holder 6, through which a syringe or medicament container located inside the syringe holder 6 can be investigated. The windows 60 are delimited by a wall portion 60a of the syringe holder 6. The diameter of the windows 60 decreases in inward radial direction.

The syringe holder 6 further comprises snap features 62, namely ribs, protruding in outward radial direction. A respective snap feature 62 is located at the distal end and at the proximal end of the window 60. The snap features 62 are configured to engage with the housing 100 to fix the syringe holder 6 to the housing 100 such that an axial and a rotational movement of the syringe holder 6 with respect to the housing 100 is prevented.

In figure 45, the ribs 61 project into the recesses 54 allowing an axial movement of the needle shroud 5 with respect to the syringe holder 6 but preventing a rotational movement of the needle shroud 5 with respect to the syringe holder 6. For that purpose, the width of the recesses 54 might be substantially as great as the width of the ribs 61.

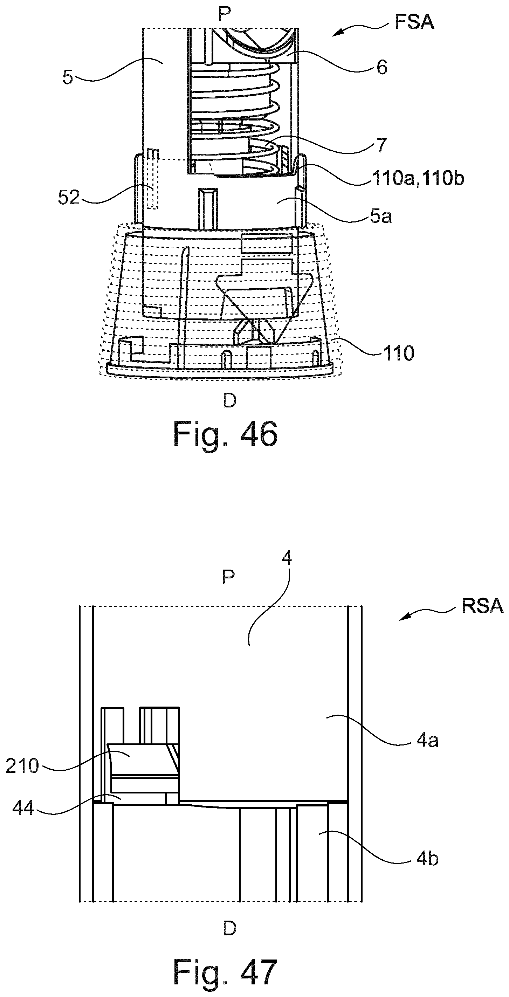

Figure 46 shows a detailed view of the distal end of the front subassembly FSA with the cap 110 attached to the needle shroud 5. The protrusions 110b of the resilient arms 110a project into the recesses 52 of the needle shroud 5 so that the cap 110 is loosely held in position with respect to the needle shroud 5.

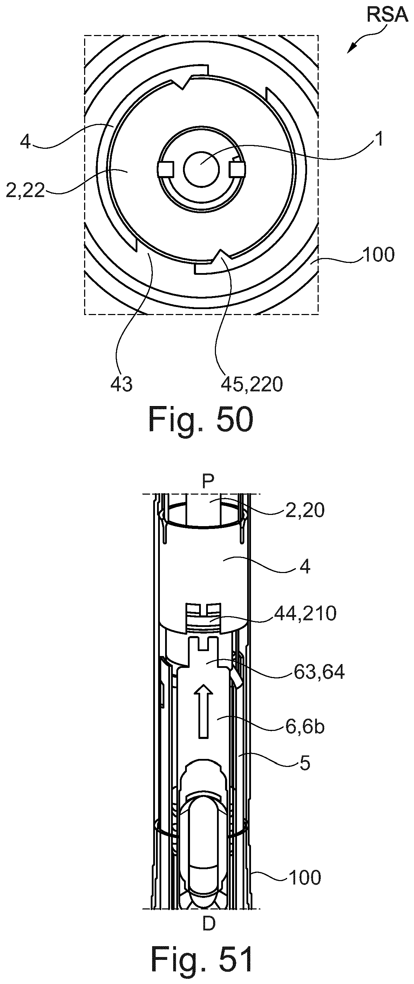

Figure 47 shows a section of the rear subassembly RSA in perspective view. Figure 48 shows the rear subassembly RSA in cross-sectional view with the longitudinal axis A running in the cutting plane. Figure 50 shows the rear subassembly RSA in a cross-sectional view with the cutting plane running perpendicularly to the longitudinal axis A. An exemplary embodiment of the second locking mechanism is illustrated on the basis of these figures.

As can be seen in figure 47, a recess 44, particularly a cut-out, is formed in the first section 4a of the syringe holder 4. The first portion 21 of the rotating collar 2 comprises a displaceable axial-lock element 210 in form of a resilient arm 210 or clip 210. The resilient arm 210 is displaceable in radial direction. The resilient arm 210 is configured to project into the recess 44 when it is in a first radial position. In this case, the rear subassembly RSA is in a second locked state. The engagement of the resilient arm 210 and the recess 44 establishes an axial-lock interface and prevents a proximal movement of the rotating collar 2 with respect to the drive spring holder 4, because, when moving the rotating collar 2 in proximal direction P, the resilient arm 210 hits against an edge of the drive spring holder 4 delimiting the recess 44 in proximal direction P. This is one part of the second locking mechanism, also referred to as axial-locking mechanism.

As can be seen in figure 48, in the second locked state, the second portion 22 of the rotating collar 2 abuts against the second bottom ring 4d of the drive spring holder 4. The first portion 21 of the rotating collar 2 abuts against the first bottom ring 4c of the drive spring holder 4.

The second locking mechanism comprises also a protrusion 45 (see also figure 49) which is part of the second portion 4b of the drive spring holder 4 protruding radially inwardly. The protrusion 45 is not movable in any direction with respect to the rest of the drive spring holder 4. The protrusion 45 may have the same form as the first section 410b of the protrusion 410 of the resilient arm 41. The protrusion 45 is offset in distal direction D with respect to the resilient arm 41 or the protrusion 410, respectively. Furthermore, the second locking mechanism comprises the second section 22 of the rotating collar 2 with the above described recess 220 forming also part of the previously described first locking mechanism.

In the second locked state, the protrusion 45 projects into the recess 220 (see figure 50) thereby establishing a rotation-lock interface. This engagement prevents a rotation of the rotating collar 2 (the biased torsion drive spring 3 may already induce a torque onto the rotating collar 2 in the second locked state). This is another part of the second locking mechanism, also referred to a second rotation-locking mechanism.

The second rotation-locking mechanism does not need the needle shroud 5 for retaining the second locked state as the protrusion 45 is not displaceable in radial direction. Thus, as long as the rotating collar 2 is not moved in proximal direction P, a rotation of the rotating collar 2 is not possible.

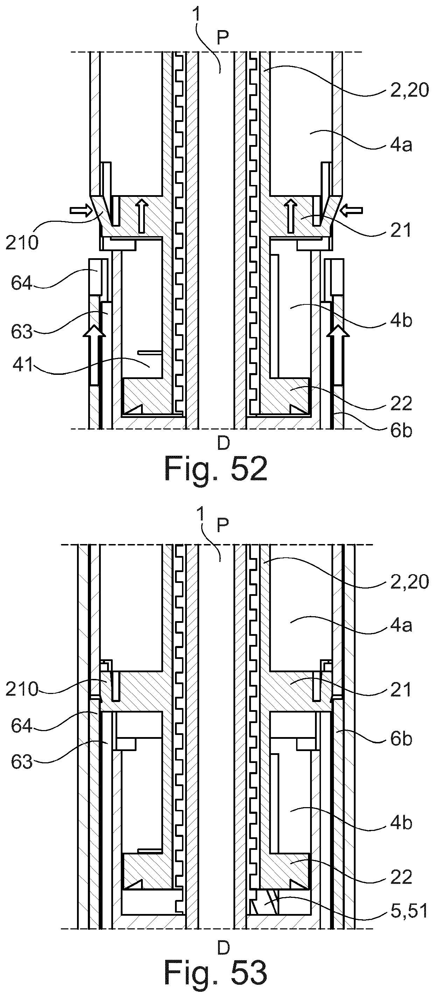

Figure 51 shows a position in the assembly of the auto-injector, in which the rear subassembly and the front subassembly of the previous figures are telescoped into each other. Figure 52 shows the same position in the assembling as figure 51 but in a cross-sectional view.

As can be seen in figure 52, the arms 6b of the syringe holder 6 each comprise or form at their proximal ends a push element 63 and a release element 64. The release element 64 protrudes beyond the push element 63 in proximal direction P. Moreover, the push element 63 is offset in inward radial direction with respect to the release element 64. When being telescoped into each other, the release element 64 first hits against the resilient arm 210 and forces the resilient arm 210 to move radially inwardly so that the axial-locking mechanism is released. This is realized by the resilient arm 210 having a beveled surface tilted with respect to the longitudinal axis so that a force acting on the beveled surface in proximal direction P pushes the resilient arm 210 in inward radial direction.

At the same time or later during telescoping the rear subassembly into the front subassembly, the push element 63 hits against the first section 21 of the rotating collar 2 and pushes the rotating collar 2 in proximal direction P (see also figure 53). This results in a release of the second rotation-locking mechanism and a transfer from the second locked state into the first locked state. The first locked state is occupied because the pushing of the rotating collar 2 in proximal direction P is accompanied with the needle shroud 5 being brought in the position where it holds the resilient arm 41 in its first radial position. Pushing the rotating collar 2 in proximal direction P during assembly has as a consequence that the recess 220 in the second portion 22 disengages with the protrusion 45 but before engages with the protrusion 410 of the resilient arm 41 (see also figure 49).

### 8. Feedback mechanism

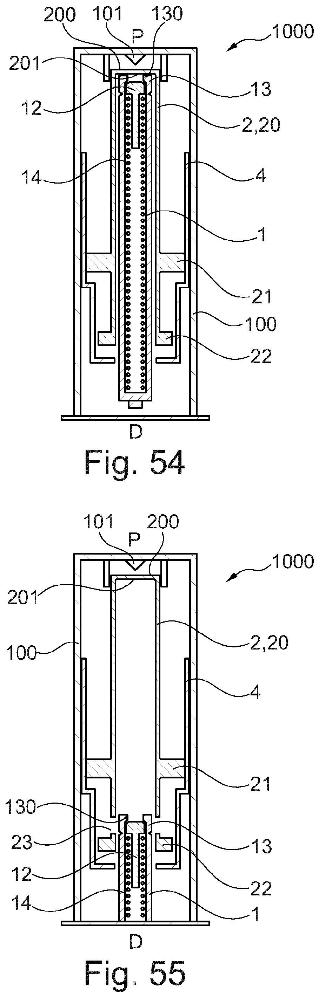

Figures 54 to 56 illustrate an exemplary embodiment of a feedback mechanism. Such a feedback mechanism can be used in any one of the exemplary embodiments of a drug delivery device described herein.

Figure 54 shows a section of an exemplary embodiment of a drug delivery device/auto-injector 1000 with such a feedback mechanism. In figure 54, the auto-injector 1000 may be in the first locked state (initial state).

The feedback mechanism comprises a plunger rod 1 received in a rotating collar 2. The rotating collar 2 may be designed as described in connection with the previous figures. Particularly, the rotating collar 2 is a sleeve. The plunger rod 1 is hollow, e.g. hollow cylindrically-shaped. A feedback energy member 14 in the form of a spring 14, e.g. compression spring, is received in the plunger rod 1, i.e. in a cavity thereof. Furthermore, a feedback element 12 in the form of a piston 12 is received in the plunger rod 1. The spring 14 is connected to the piston 12 and to the plunger rod 1 and is compressed. The spring 14 induces a force onto the piston 12 pointing in proximal direction P, i.e. the piston 12 is biased in proximal direction P relative to the plunger rod 1.

The plunger rod 1 comprises displaceable arms 13 oriented in axial direction. The displaceable arms 13 may be resilient arms 13 and are located at the proximal end of the plunger rod 1. The displaceable arms 13 each comprise a stop feature 130 in the form of a protrusion 130 at their respective proximal end. The displaceable arms 13 together with their protrusions 130 are each displaceable in radial direction. The displaceable arms 13 are each in a first radial position. They may be biased in the outward radial direction. However, the displaceable arms 13 are held in the first radial position by a sidewall of the rotating collar 2 circumferentially surrounding the plunger rod 1 at least at the height of the displaceable arms 13.

The protrusions 130 of the displaceable arms 13 project into the cavity of the plunger rod 1. The proximal end of the piston 12 abuts against the protrusions 130. This prevents a movement of the piston 12 in proximal direction P driven by the spring 14 beyond the protrusions 130.

As visible in figure 54, the piston 12 and the protrusions 130 each comprise slide features in the form of beveled surfaces tilted with respect to the longitudinal axis and the radial direction. The piston 12 and the protrusions 130 abut against each other at the beveled surfaces which biases the protrusions 130 or the displaceable arms 13, respectively, in outward radial direction.

Figure 55 shows the auto-injector 1000 in the released state. The torsion drive spring induces a torque onto the rotating collar 2 which starts rotating in a first rotational direction and thereby the plunger rod 1 is moved in distal direction D. The biased spring 14 and the piston 12 move together with the piston rod 1 in distal direction D. During the movement, the displaceable arms 13 of the plunger rod 1 are held in the first radial position by the sidewall of the rotating collar 2 still circumferentially surrounding the resilient arms 13.

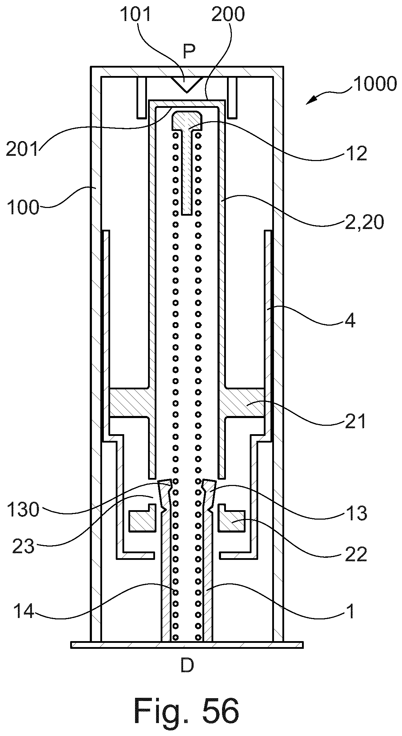

In a region of the distal end of the rotating collar 2, namely in the region between the first section 21 and the second section 22, the side wall of the rotating collar 2 is interrupted by a recess 23. When the plunger rod 1 reaches a feedback position, the displaceable arms 13 or the protrusions 130, respectively, axially and rotationally overlap with this recess 23. Thus, the displaceable arms 13 are no longer held in the first radial position. As they are biased radially outwardly, the displaceable arms 13 leave the first radial position and move in outward radial direction into a second radial position. In the second radial position, the piston 12 is no longer prevented from moving in proximal direction P relative to the plunger rod 1 driven by the spring 14 beyond the protrusions 130. This is illustrated in figure 56.

In figure 56, it can be seen that the piston 12, due to the force induced by the spring 14, moves in proximal direction P, thereby leaves the plunger rod 1 and finally hits against a proximal end 201 of the rotating collar 2 forming an impact feature 201. This hit may cause an audible and/or tactical feedback which indicates the user the end of the drug delivery process. For example, the auto-injector is designed such that the piston 12 hitting the impact feature 201 creates a noise of at least 20 dB.

### 9. Third exemplary embodiment of a drug delivery device

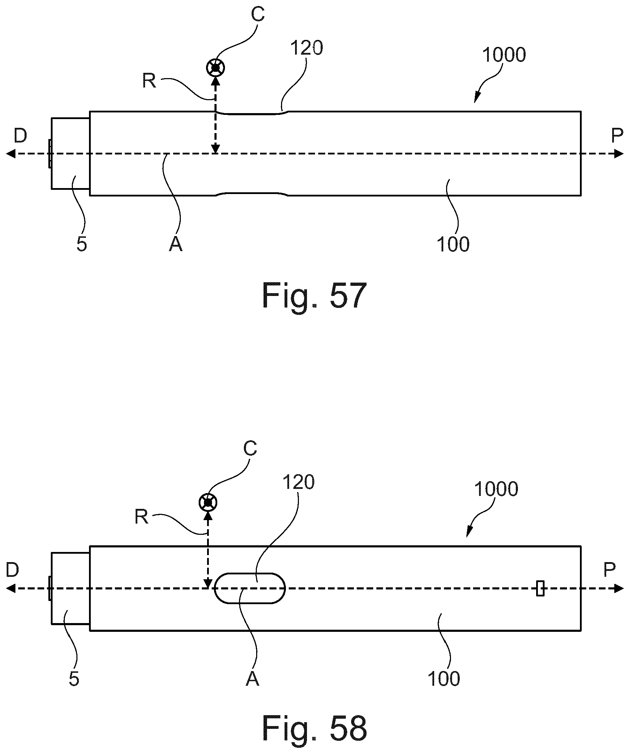

Figures 57 and 58 show a third exemplary embodiment of a drug delivery device 1000. Figure 57 is a side view and figure 58 is a side view rotated by 90° around the longitudinal axis A with respect to figure 57. The drug delivery device 1000 is an auto-injector.

The auto-injector 1000 comprises a housing 100 with a window 120. The window 120 may be used for inspecting the fill level of a medicament container or a syringe or a progress of a stopper inside the housing 100 or the drug clarity or the degradation of the drug.

The auto-injector 1000 further comprises a protection member 5 in the form of a needle shroud 5 which is telescopically coupled to the housing 100 and is axially movable with respect to the housing 100.

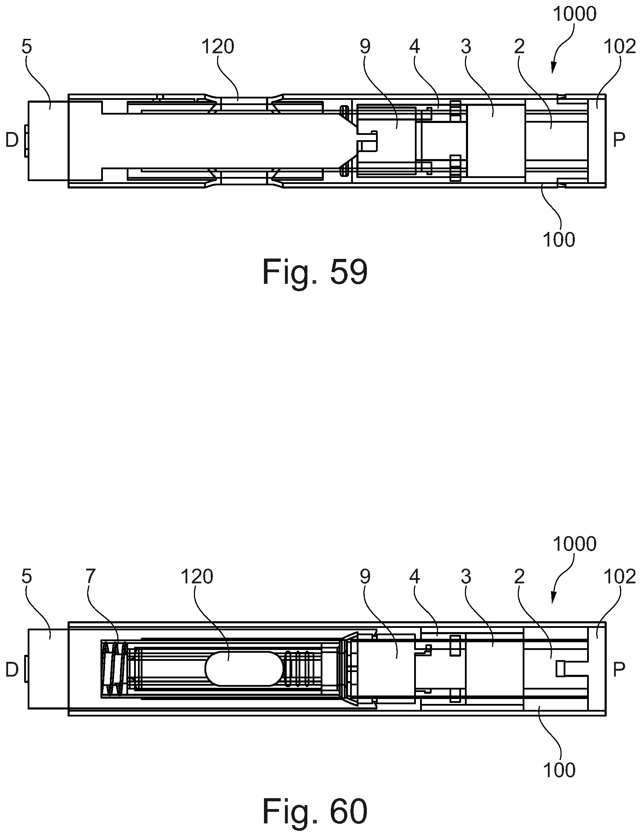

Figures 59 and 60 show the auto-injector 1000 of figures 57 and 58 in the same views but now with the housing 100 being indicated semi-transparent which allows to see further members and elements of the auto-injector 1000. It can be seen, that the auto-injector 1000 further comprises a rear cap 102 which closes the housing 100 at the proximal end. Furthermore, the auto-injector 1000 comprises a drive spring holder 4, which is hollow, e.g. a sleeve. A torsion drive spring 3 is received in the drive spring holder 4. The torsion drive spring may be a spiral torsion spring. A rotating collar 2 is received in the torsion drive spring 3 and the drive spring holder 4. Moreover, a moveable member 9, also referred to as activation element 9, in the form of an activation collar 9 is provided. The activation collar 9 is releasably axially coupled to the needle shroud 5 so that an axial movement of the needle shroud 5 induces an axial movement of the activation collar 9. The activation collar 9 is located downstream of the torsion drive spring 3 in distal direction D and circumferentially surrounds a portion of the rotating collar 2.

Furthermore, the auto-injector 1000 comprises a shroud spring 7 which couples the needle shroud 5 to the housing 100. The coupling via the shroud spring 7 is such that a proximal movement of the needle shroud 5 with respect to the housing 100 compresses the shroud spring 7. This compression biases the needle shroud 5 in distal direction D relative to the housing 100.

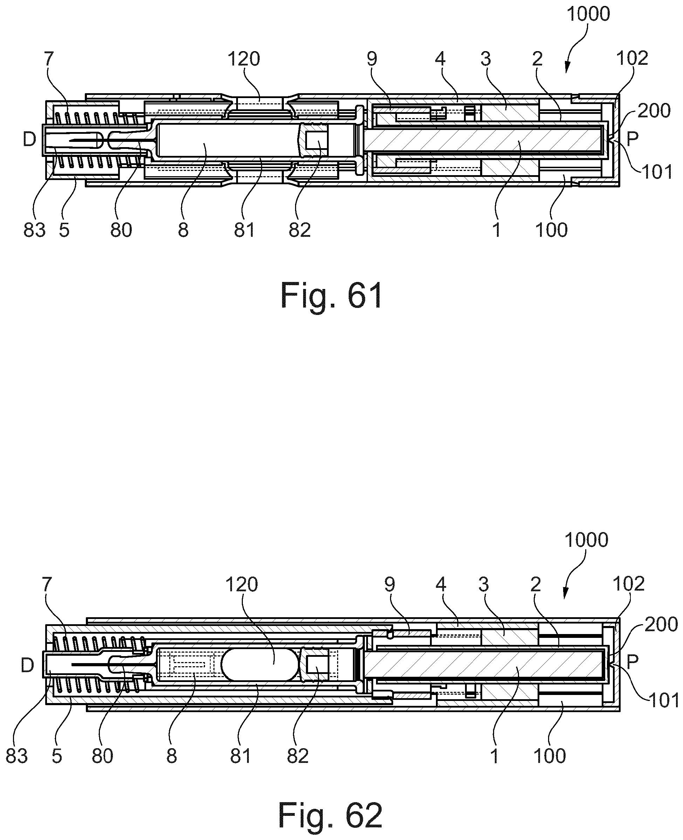

Figure 61 and 62 show the auto-injector 1000 of figures 57 and 58 in the same views but now in a cross-sectional view with the cutting plane comprising the longitudinal axis A. In this view, it can be seen that the auto-injector 1000 further comprises a plunger rod 1. The plunger rod 1 is to a main part received in the rotating collar 2 and is circumferentially surrounded by the rotating collar 2. Only a small portion of the plunger rod 1 (less than 50 % of its length) projects out of the rotating collar 2 in distal direction D. In proximal direction P, the rotating collar 2 is closed and the plunger rod 1 does not project beyond the proximal end of the rotating collar 2. The plunger rod 1 is longer, measured along the longitudinal axis, than the rotating collar 2.

The housing 100, the housing element 4, the plunger rod 1, the rotating collar 2, the needle shroud 5 and the activation element 9 may all comprise or consist of plastic. All these members may each be formed in one piece. The drive spring 3 and the shroud spring 7 may comprise or consist of a metal, e.g. steel.

It can be seen in figures 61 and 62 that a medicament container 8, in the present case a syringe 8, is arranged in the housing 100. This syringe 8 may be arranged axially and/or rotationally and/or radially fixed with respect to the housing 100. The syringe 8 comprises a cartridge 81 filled with a drug, a needle 80 and a stopper 82. The needle 80 is arranged at a distal end of the syringe 8. The stopper 82 seals the cartridge 81 in proximal direction P. When moving the stopper 82 in the distal direction D, the drug stored in the cartridge 81 is pressed out of the syringe 8 through the needle 80.

In figures 61 and 62 it can be further seen that the needle 80 is covered by a needle shield 83 which encapsulates the needle 80 and projects beyond the needle 80 in distal direction D. The needle shield 83 may be removed before using the auto-injector 1000.

For using the auto-injector 1000, the distal end of the auto-injector 1000 formed by the needle shroud 5 may be pressed against a body, e.g. a human body. As a consequence of that, the needle shroud 5 moves from its extended position in the proximal direction P with respect to the housing 100. This results in the needle 80 being exposed and projecting in distal direction D so that it can now pierce into the tissue of the body.

In the position shown in figure 61 and 62, the auto-injector 1000 is still in an initial state, in the following referred to as locked state, in which the torsion drive spring 3 is biased and induces a torque onto the rotating collar 2. A locking mechanism, however, prevents the rotating collar 2 from a rotational movement. The locking mechanism will be explained in more detail further below.

In the locked state, a proximal end of the rotating collar 2 may be axially spaced from a proximal end-stop of the housing 100. This allows an axial movement of the rotating collar 2 in proximal direction P. Moreover, in the locked state, a distal end of the plunger rod 1 is axially spaced from the stopper 82 of the syringe 8. Thus, the plunger rod 1 can axially move in the distal direction D for a predetermined distance before hitting the stopper 82.

The needle shroud 5 may be moved in the proximal direction P into a retracted position. This releases the locking mechanism so that the rotating collar 2 is no longer prevented from rotating. The auto-injector switches from the locked state into a released state. The torque induced by the torsion drive spring 3 onto the rotating collar 2 forces the rotating collar 2 to rotate in a first rotational direction (clockwise or counterclockwise). For example, the rotating collar 2 rotates several times around its rotational axis. A drive mechanism, e.g. the drive mechanism described before, converts the rotation of the rotating collar 2 into an axial movement of the plunger rod 1 in the distal direction D. After having moved the predetermined distance in the distal direction D, the plunger rod 1 hits the stopper 82 of the syringe 8 and can now push the stopper 82 in distal direction D which results in the drug in the cartridge 81 being pressed out through the needle 80 into the tissue.

The rotating collar 2 may not only rotate but also moves in proximal direction P until the proximal end of the rotating collar 2 hits the proximal end-stop of the housing 100. The end-stop comprises is a protrusion 101 which tapers in distal direction D. The protrusion 101 may be a cone. The proximal end of the rotating collar 2 comprises an indentation 200. For example, the surface of the proximal end of the rotating collar 2 is concavely shaped. The protrusion 101 can penetrate into the indentation 200 when the proximal end of the rotating collar 2 hits the end-stop of the housing 100. The protrusion 101 and the indentation 200 may each be designed rotationally symmetric or circular symmetric with respect to the rotational axis of the rotating collar 2. In this way, a low friction interface is formed between the housing 100 and the rotating collar 2 so that a low friction rotation of the rotating collar 2 is enabled also when the proximal end of the rotating collar 2 abuts against the housing 100. Particularly, the radius at which the friction between the rotating collar 2 and the end-stop acts is approaching zero or is zero, therefore the resulting torque from the friction also tends to zero significantly reducing losses allowing a reduced spring force and/or enhance injection performance.

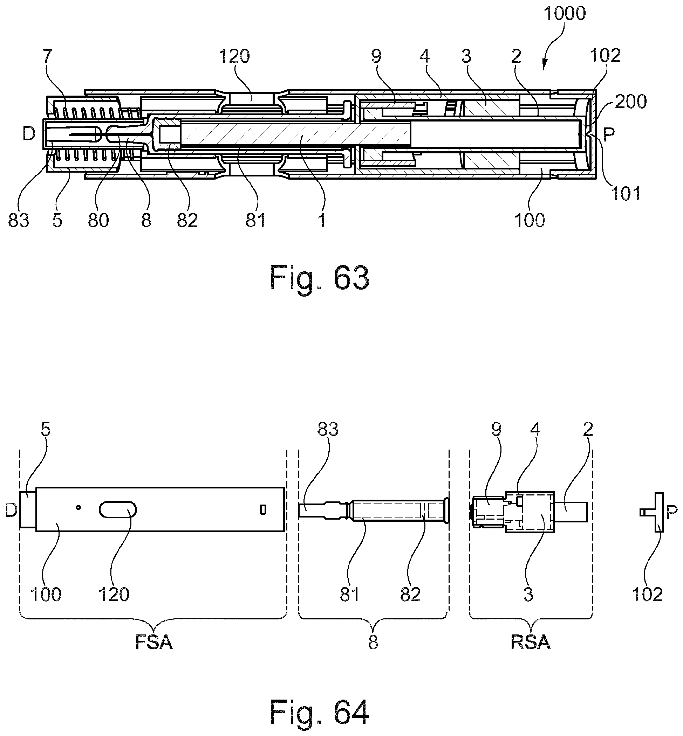

Figure 63 shows the auto-injector 1000 according to the third exemplary embodiment in a cross-sectional view and after usage. The plunger rod 1 has hit the stopper 82 and has pushed the stopper 82 into distal direction D. As a consequence, the drug in the cartridge 82 was pushed through the needle 80 out of the syringe 8. For example, the drug was thereby injected into the tissue of the body.

Figure 64 shows different subassemblies of the auto-injector 1000 according to the third exemplary embodiment. The auto-injector 1000 comprises a front subassembly FSA. The front subassembly FSA comprises the housing 100, the needle shroud 5 and the shroud spring 7 coupling the housing 100 and the needle shroud 5.

The auto-injector 1000 further comprises a rear subassembly RSA, with the plunger rod 1, the rotating collar 2, the torsion drive spring 3, the drive spring holder 4 and the activation collar 9.

When assembling the front subassembly FSA and the rear subassembly RSA, a syringe 8 is first telescoped into the housing 100 of the front subassembly FSA and then the rear subassembly RSA is telescoped into the housing 100. Finally the rear cap 102 is attached to the proximal end of the housing 100 and may be fixed to the housing 100 via a clip.

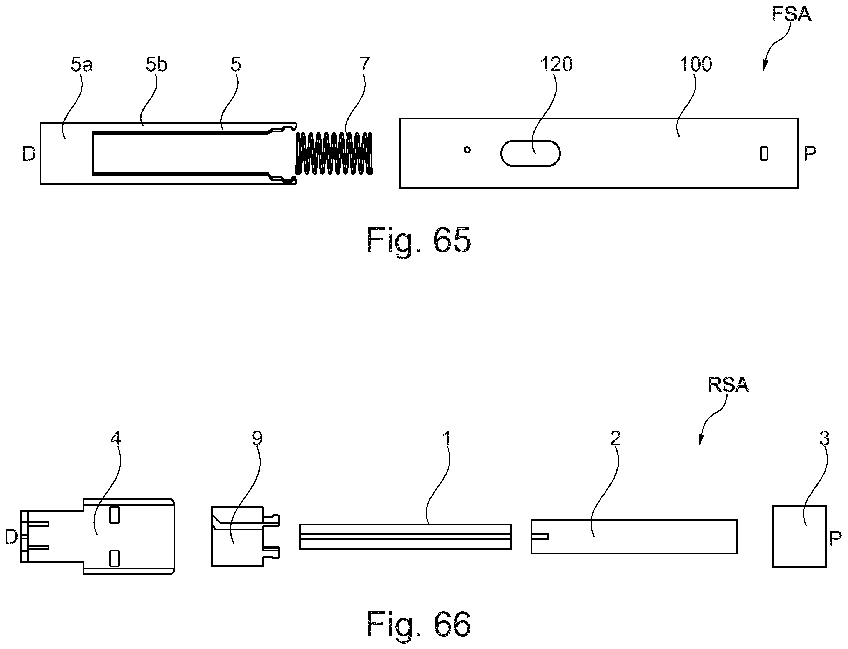

Figure 65 shows the front subassembly FSA in an exploded view. The needle shroud 5 comprises a distal portion 5a which is shaped hollow cylindrically and into which the shroud spring 7 can be telescoped. Furthermore, the needle shroud 5 comprises two arms 5b extending from the cylindrically shaped portion 5a in proximal direction P.

Figure 66 shows the rear subassembly RSA in an exploded view.

### 9.1 Drive mechanism of the drug delivery device according to the third exemplary embodiment

The drive mechanism of the auto-injector according to the third exemplary embodiment may be designed as the previously described drive mechanism.

### 9.2 Locking mechanism of the drug delivery device according to the third exemplary embodiment

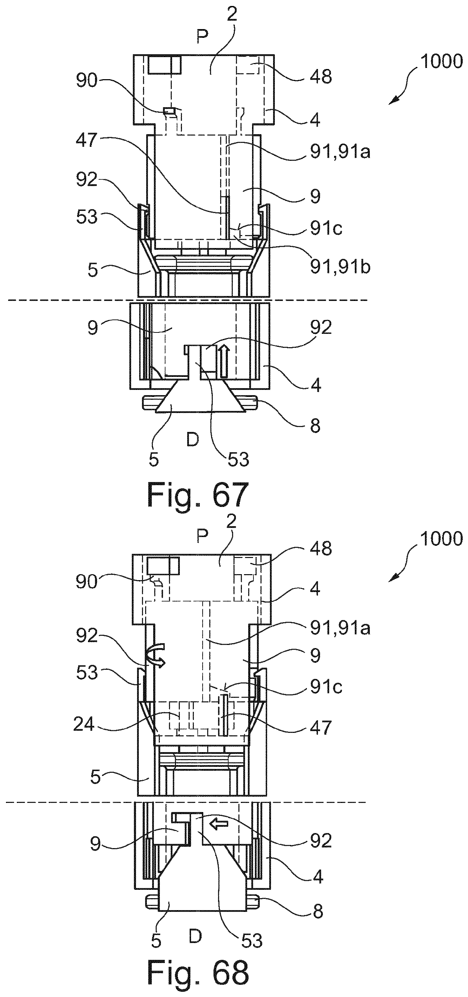

Figure 67 shows sections of the auto-injector 1000 according to the third exemplary embodiment in the locked state.

The upper part of figure 67, above the horizontal dashed line, shows a section of the auto-injector 1000 in a side view. The lower part of figure 67, below the dashed line, shows a section of the auto-injector in a side view rotated by 90° around the longitudinal axis A with respect to the upper part.

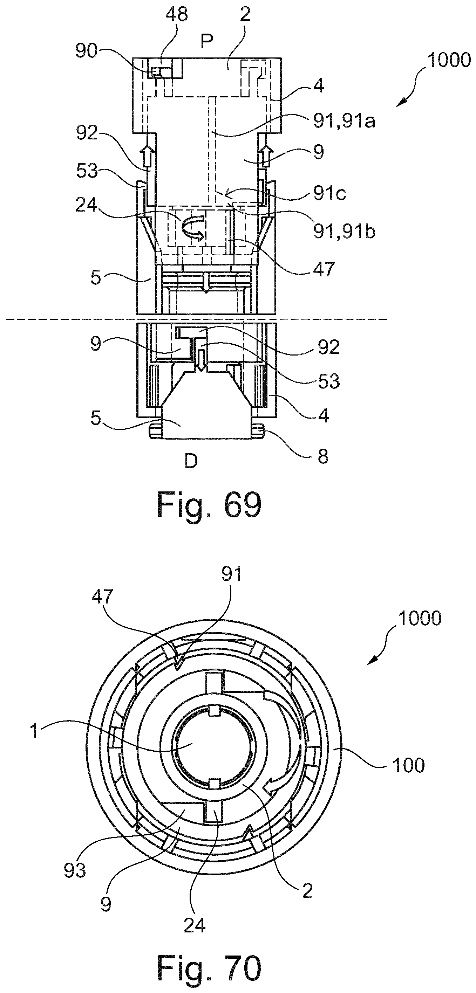

Figure 70 shows the auto-injector 1000, e.g. also in the locked state, in a cross-sectional view with the crossing plane being perpendicular to the longitudinal axis A.

Considering first figure 67, the needle shroud 5 comprises a coupling feature 53 in the form of a resilient arm 53 with a protrusion projecting radially inwardly. The activation collar 9 has a coupling feature 92 in the form of a recess 92 or opening 92. The protrusion of the resilient arm 92 projects into the recess 92. In this way, the needle shroud 5 and the activation collar 9 are axially coupled so that an axial movement of the needle shroud 5 induces an axial movement of the activation collar 9.

In the lower part of figure 67 it can be seen that the recess 92 is L-shaped and comprises two sections being adjacent to each other in the angular direction. In the locked state shown in figure 67, the resilient arm 53 engages into a first section of the recess 92. The first section of the recess 92 is bordered in proximal direction P and distal direction D by edges of the activation collar 9. Thus, an axial movement of the needle shroud 5 in proximal direction P and distal direction D results in the protrusion of the resilient arm 53 hitting either one of these edges. As a consequence, the activation collar 9 is forced to move in distal direction D when the needle shroud 5 is moved in distal direction D and the activation collar 9 is forced to move in proximal direction P when the needle shroud 5 is moved in proximal direction P. In other words, the needle shroud 5 is coupled to the activation collar 9 in proximal direction P and distal direction D.

On the other hand, the second section of the recess 92 is delimited by an edge of the activation collar 9 only in proximal direction P. In distal direction D, the second section of the recess 92 is open and not delimited by an edge of the activation collar 9. Thus, if the protrusion of the resilient arm 53 would engage into the second section of the recess 92, the protrusion would hit against an edge of the activation collar 9 when moving the needle shroud 5 in proximal direction P which would force the activation collar 9 to also move in proximal direction P. A movement of the needle shroud 5 in distal direction D, however, would result in a disengagement of the resilient arm 53 and the recess 92.

Furthermore, it can be seen in figure 67 that the activation collar 9 is coupled to the drive spring holder 4 via a first rotation-lock interface. The first rotation-lock interface prevents a rotation of the activation collar 9 with respect to the drive spring holder 4. On the other hand, as can be seen in figure 70, the rotating collar 2 and the activation collar 9 are coupled via a second rotation-lock interface. The second rotation-lock interface prevents a rotation of the rotating collar 2 with respect to the activation collar 9. Thus, in sum, a rotation of the rotating collar 2 with respect to the drive spring holder 4 is prevented by the two rotation-lock interfaces.

The first rotation-lock interface is established by a slit 91a in the activation collar 9 and a rib 47 of the drive spring holder 4 engaging into the slit 91. The rib 47 and the slit 91 are each elongated with a main extension direction along the longitudinal axis. As can be seen in figure 67, the slit 91a is a first section of a recess 91 in the activation collar 9. The recess 91 also comprises a second section 91b adjoining the slit 91a in distal direction D. The slit 91 has a smaller width, measured along the angular direction, than the second section 91b. The width of the second section 91b first increase in direction away from the slit 91a and then has a constant width. In this region of increasing width, the second section 91b is delimited by a beveled surface 91c of the activation collar 9 which is tilted with respect to the longitudinal axis and the rotational direction. This beveled surface 91c realizes a slide feature. In the locked state, shown in figure 67, the rib 47 engages into the slit 91a of the recess 91.

As can be seen in figure 70, the second rotation-lock interface is realized by a protrusion 93 of the activation collar 9 and a protrusion 24 of the rotating collar 2 abutting against each other in angular direction. The protrusion 93 of the activation collar 9 projects radially inwardly and the protrusion 24 of the rotating collar 2 projects radially outwardly. The protrusions 24, 93 abut against each other such that a rotation of the rotating collar 2 induced by the biased torsion drive spring 3 with respect to the activation collar 9 is prevented or blocked by the activation collar 9.

Figure 68 shows the autoinjector 1000 in a position in which the needle shroud 5 has been moved from its extended position proximally towards the retracted position. The needle shroud 5 is now in an intermediate position between the extended position and the retracted position. In this intermediate position, the rib 47 is transferred from the slit into the second section 91b. Due to the force induced by the drive spring 3, the beveled surface 91c is pressed against the rib 47 and the rib 47 slides along the beveled surface 91c whereby the activation collar 9 rotates with respect to the drive spring holder 4 and with respect to the needle shroud 5 by a predetermined angle in the first rotational direction. This rotation happens automatically, as the torque induced by the torsion drive spring 3 is transferred via the rotating collar 2 to the activation collar 9 (via the second rotation-lock interface). After the rotation by the predetermined angle, an edge of the activation collar 9 running parallel to the longitudinal axis and delimiting the second section 91b of the recess 91 in angular direction hits against the rib 47. A further rotation of the activation collar 9 with respect to the drive spring holder 4 in the first rotational direction is then prevented.

However, the rotation of the activation collar 9 by the predetermined angle in the first rotational direction has as a consequence that the resilient arm 53 of the needle shroud 5 now engages into the second section of the recess 92 of the activation collar 9 which results in a decoupling of the activation collar 9 and the needle shroud 5in distal direction D. In other words, the coupling of the needle shroud 5 and the activation collar 9 in distal direction D is released.

Figure 69 shows the auto-injector 1000 in a position in which the needle shroud 5 has been further moved in proximal direction P into the retracted position which has also forced the activation collar 9 to further move in proximal direction P. In this retracted position of the needle shroud 5, the needle 80 of the auto-injector 1000 may be exposed allowing the needle 80 to pierce into a tissue of a body. In the retracted position of the needle shroud 5, the second rotation-lock interface between the activation collar 9 and the rotating collar 2 is released, i.e. the protrusion 24 and the protrusion 93 are now axially offset and do not abut against each other any longer so that the auto-injector 1000 transfers into the released state in which the rotation of the rotating collar 2 with respect to the activation collar 9 and with respect to the drive spring holder 4 is enabled. The rotating collar 2 rotates in the first rotational direction and thereby drives the plunger rod 1 in distal direction D which results in a delivery of the drug through the needle 80 (see description above).

Furthermore, the movement of the activation collar 9 further in proximal direction P had as a consequence that a second coupling feature 90 of the activation collar 9, namely a clip 90, has engaged into a coupling feature 48 of the drive spring holder 4, namely a recess 48. The engagement between the clip 90 and the recess 48 is such that a movement of the activation collar 9 in distal direction D is prevented. When moving the needle shroud 5 from the retracted position back towards or into the extended position, the activation collar 9 does not and cannot follow. A movement of the needle shroud 5 in distal direction D relative to the activation collar 9 is enabled since the resilient arm 53 engages into the second section of the recess 92 as described above.

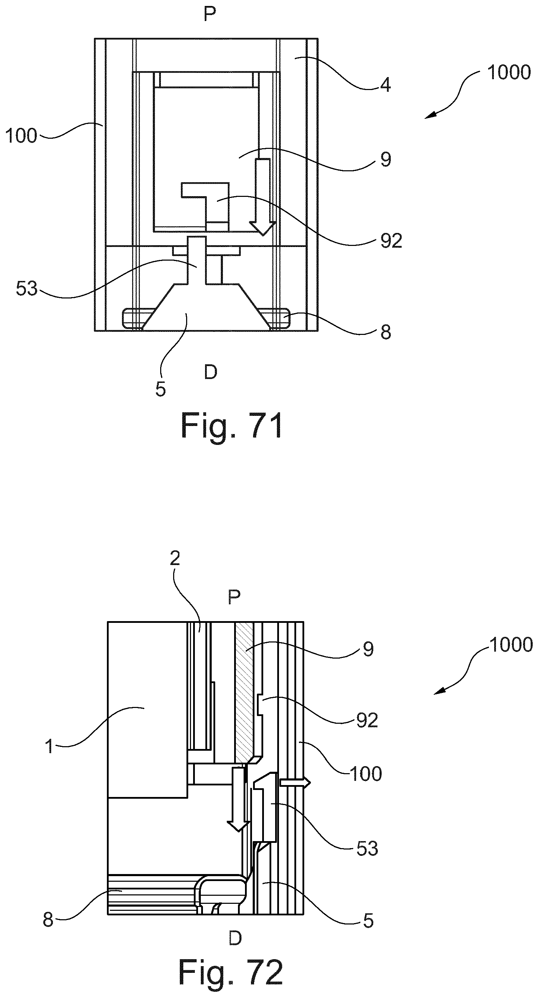

Figure 71 to 73 show different positions during assembling the auto-injector 1000 according to the third exemplary embodiment. The rear subassembly is telescoped into the front subassembly.

Figure 71 shows a first position, in which the needle shroud 5 of the front subassembly and the activation collar 9 of the rear subassembly are not yet coupled to each other. Figure 71 is a side view of the auto-injector 1000 during assembling.

Figure 72 shows the position of figure 71 in a cross-sectional view. It can be seen, that the resilient arm 53 of the needle shroud 5 has a slide feature in form of a beveled surface. The beveled surface is designed such that, when the beveled surface hits the distal end of the activation collar 9, a force is created pushing the resilient arm 53 in outward radial direction. The rear subassembly and the front subassembly can then further be telescoped into each other and as soon as the protrusion of the resilient arm 53 axially and rotationally overlaps with the recess 92 of the activation collar 9, it slips into this recess 92. In this way, a coupling between the activation collar 9 and needle shroud 5 is obtained.

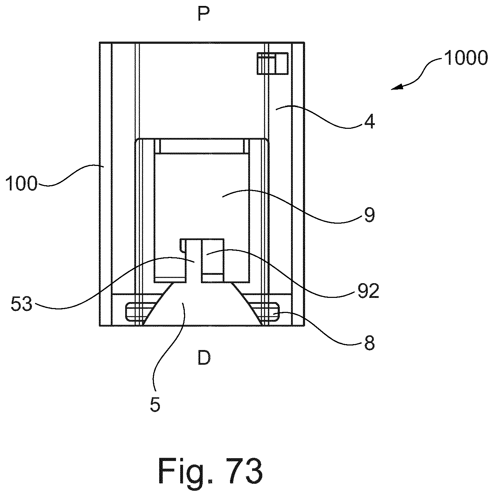

Figure 73 shows the auto-injector after coupling of the needle shroud 5 and the activation collar 9.

### Further explanations and definitions

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide. Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

The invention described herein is not limited by the description in conjunction with the exemplary embodiments beyond the scope as defined by the appended claims. Rather, the invention comprises any new feature as well as any combination of features, particularly including any combination of features in the patent claims, even if said feature or said combination per se is not explicitly stated in the patent claims or exemplary embodiments where the scope is defined by the appended claims.

### Reference numerals

- 1: plunger rod
- 2: rotating collar
- 3: torsion drive spring
- 4: drive spring holder
- 4a: first section of drive spring holder 4
- 4b: second section of drive spring holder 4
- 4c: first bottom ring of drive spring holder 4
- 4d: second bottom ring of drive spring holder 4
- 5: needle shroud
- 5a: cylindrically-shaped portion
- 5b: arm
- 6: medicament container holder/ syringe holder
- 6a: cylindrically-shaped portion
- 6b: arm
- 6c: support portion
- 7: shroud spring
- 8: medicament container/syringe
- 9: activation collar
- 10: groove
- 11: external thread
- 12: piston
- 13: displaceable arm
- 14: feedback energy member/ spring
- 20: shaft
- 21: first portion
- 22: second portion
- 22a: surface
- 23: recess
- 24: protrusion
- 40: protrusion
- 40a: edge in drive spring holder 4
- 41: resilient arm
- 43: recess
- 44: recess
- 45: protrusion
- 46: flap
- 47: rib
- 48: recess
- 50a: wall portion
- 50b: recess
- 51: resilient arm
- 51a: ramp
- 52: recess
- 53: resilient arm
- 54: recess
- 60: window
- 60a: wall portion
- 61: rib
- 62: snap feature
- 63: push element
- 64: release element
- 80: needle
- 81: cartridge
- 82: stopper
- 83: needle shield
- 90: clip
- 91: recess
- 91a: slit/first section of recess 91
- 91b: second section of recess 91
- 91c: tilted surface
- 92: recess
- 93: protrusion
- 100: housing
- 101: protrusion
- 102: rear cap
- 110: cap
- 110a: resilient arm
- 110b: protrusion
- 111: grabber
- 120: window
- 130: protrusion
- 200: indentation
- 201: impact feature
- 210: resilient arm/ clip
- 220: recess
- 220b: first section of recess 220
- 220c: second section of recess 220
- 220d: surface of recess 220
- 221: recess
- 221a: beveled surface
- 410: protrusion
- 410a: beveled surface
- 410b: first section of protrusion 410
- 410c: second section of protrusion 410
- 410d: surface of protrusion 410
- 411: protrusion
- 411a: beveled surface
- 1000: drug delivery device / auto-injector
- FSA: front sub assembly
- RSA: rear sub assembly
- α: angle
- D: distal direction
- P: proximal direction
- A: longitudinal axis/ axial direction
- R: radial direction
- C: azimuthal/rotational/angular direction

## Claims

1. Arrangement for a drug delivery device (1000) comprising:
- a housing element (4),
- a plunger rod (1) being arranged axially movable with respect to the housing element (4),
- a feedback element (12) being arranged axially movable with respect to the housing element (4) and with respect to the plunger rod (1),
- a feedback energy member (14) configured to provide energy in order to induce a movement of the feedback element (12) relative to the plunger rod (1) in a first axial direction,
- a radially displaceable stop feature (130) and an impact feature (201), wherein
- the arrangement has an initial state, in which
- the plunger rod (1) is in a proximal position,
- the feedback element (12) is arranged in a cavity of the plunger rod (1),
- the feedback element (12) is limited in its movement relative to plunger rod (1) in the first axial direction by the stop feature (130) being in a first radial position,
- the arrangement is configured to be switched from the initial state into a released state, in which
- the plunger rod (1) is moved in a distal direction (D) at least until the plunger rod (1) reaches a feedback position,
- the movement of the plunger rod (1) into the feedback position results in the stop feature (130) being radially displaced to enable a movement of the feedback element (12) relative to the plunger rod (1) in the first axial direction beyond the stop feature (130),
- the feedback element (12) moves relative to the plunger rod (1) in the first axial direction and beyond the stop feature (130) due to the energy provided by the feedback energy member (14) until the feedback element (12) hits against the impact feature (201), thereby producing a tactile and/or audible feedback, **characterised in that**
- the stop feature (130) is axially and/or rotationally fixed to the plunger rod (1).

2. Arrangement according to claim 1, wherein
- the plunger rod (1) is hollow cylindrically-shaped,
- in the initial state, the feedback element (12) is completely arranged inside the plunger rod (1).

3. Arrangement according to any one of the preceding claims, wherein
- the impact feature (201) is arranged moveable with respect to the housing element (4),
- in the released state, the impact feature (201) moves with respect to the housing element (4).

4. Arrangement according to any one of the preceding claims, wherein
- the stop feature (130) is axially and rotationally fixed to the plunger rod (1),
- in the initial state, the stop feature (130) radially projects into the cavity of the plunger rod (1).

5. Arrangement according to any one of the preceding claims, further comprising
- an energy member (3) configured to provide energy in order to induce an axial movement of the plunger rod (1) in the distal direction (D), wherein
- in the initial state,
- the plunger rod (1) is coupled to the housing element (4) via a lock interface which prevents an axial movement of the plunger rod (1) induced by the energy member (3),
- in the released state,
- the lock interface is released so that an axial movement of the plunger rod (1) induced by the energy member (3) is enabled,
- the plunger rod (1) moves in distal direction (D) due to the energy provided by the energy member (3).

6. Arrangement according to claim 5, further comprising
- a transfer member (2) being arranged rotatably with respect to the housing element (4),
- wherein the transfer member (2) and the plunger rod (1) are operatively coupled such that a rotation of the transfer member (2) in a first rotational direction is converted into a movement of the plunger rod (1) in distal direction (D),
- wherein in the released state,
- the energy member (3) induces a torque onto the transfer member (2) due to which the transfer member (2) rotates in the first rotational direction and thereby forces the plunger rod (1) to move axially in distal direction (D).

7. Arrangement according to claim 6, wherein
- in the initial state, the displaceable stop feature (130) is held in the first radial position by the transfer member (2).

8. Arrangement according to claim 7, wherein
- the transfer member (2) comprises a side wall which holds the displaceable stop feature (130) in the first radial position when the arrangement is in the initial state,
- a recess (23) is formed in the side wall of the transfer member (2), wherein the recess (23) is aligned with the stop feature (130) in axial direction when the plunger rod (1) is in the feedback position so that the displaceable stop feature (130) can radially displace towards or into the recess (23).

9. Arrangement according to any one of claims 6 to 8, wherein
- the arrangement is configured such that, in the released state, the transfer member (2) moves axially with respect to the housing element (4) until it hits an end-stop of the arrangement.

10. Arrangement according to any one of the preceding claims, wherein
- the feedback energy member (14) is a spring,
- in the initial state,
- the feedback energy member (14) is arranged in the cavity of the plunger rod (1) and biases the feedback element (12) in the first axial direction,
- the feedback element (12) abuts against the stop feature (130) in the first axial direction.

11. Arrangement according to any one of the preceding claims, wherein
- the stop feature (130) is a protrusion of a displaceable arm (13),
- the displaceable arm (13) is oriented axially,
- the stop feature (130) is arranged in the region of a proximal end of the plunger rod (1),
- at least one of the stop feature (130) and the feedback element (12) comprises a slide feature against which the other element can abut and along which the other element can slide for displacing the stop feature (130) in radial direction.

12. Arrangement according to any one of the preceding claims, wherein
- in the initial state, the displaceable stop feature (130) being in the first radial position is biased in a radial direction,
- when the plunger rod (1) reaches the feedback position, the displaceable stop feature (130) automatically moves out of the first radial position into a second radial position.

13. Arrangement according to any one of the preceding claims, wherein
- in the released state, the distance the feedback element (12) moves in the first axial direction induced by the feedback energy member (14) is at least the distance between the proximal position and the feedback position of the plunger rod (1).

14. Arrangement according to any one of the preceding claims, wherein
- the feedback element (12) hitting the impact feature (201) creates an audible feedback of at least 20 dB.

15. Drug delivery device (1000) comprising:
- the arrangement according to any one of the preceding claims,
- a housing (100) with the housing element (4) fixed to or integrated in the housing (100),
- a medicament container (8) with a needle (80), the medicament container (8) comprising a medicament,
- a needle shroud (5) telescopically coupled to the housing (100) and axially movable with respect to the housing (100) between an extended position, in which the needle (80) is covered by the needle shroud (5), and a retracted position, in which the needle (80) is exposed, wherein
- the drug delivery device (1000) is configured to be switched from the initial state into the released state by moving the needle shroud (5) from the extended position into the retracted position.

## Patentansprüche

1. Anordnung für eine Medikamenten-Verabreichungsvorrichtung (1000), aufweisend:
- ein Gehäuseelement (4),
- eine Kolbenstange (1), die bezüglich des Gehäuseelements (4) axial beweglich angeordnet ist,
- ein Feedback-Element (12), das bezüglich des Gehäuseelements (4) und bezüglich der Kolbenstange (1) axial beweglich angeordnet ist,
- ein Feedback-Energieglied (14), das dazu ausgestaltet ist, Energie bereitzustellen, um eine Bewegung des Feedback-Elements (12) relativ zu der Kolbenstange (1) in einer ersten axialen Richtung zu induzieren,
- ein radial verschiebbares Anschlagmerkmal (130) und ein Aufprallmerkmal (201), wobei
- die Anordnung einen Anfangszustand hat, in dem
- die Kolbenstange (1) in einer proximalen Position ist,
- das Feedback-Element (12) in einem Hohlraum der Kolbenstange (1) angeordnet ist,
- das Feedback-Element (12) in seiner Bewegung relativ zu der Kolbenstange (1) in der ersten axialen Richtung dadurch begrenzt ist, dass das Anschlagmerkmal (130) in einer ersten radialen Position ist,
- die Anordnung dazu ausgestaltet ist, aus dem Anfangszustand in einen freigegebenen Zustand umgeschaltet zu werden, in dem
- die Kolbenstange (1) mindestens so weit in eine distale Richtung (D) bewegt wird, bis die Kolbenstange (1) eine Feedback-Position erreicht,
- die Bewegung der Kolbenstange (1) in die Feedback-Position dazu führt, dass das Anschlagmerkmal (130) radial verschoben wird, um eine Bewegung des Feedback-Elements (12) relativ zu der Kolbenstange (1) in der ersten axialen Richtung über das Anschlagmerkmal (130) hinaus zu ermöglichen,
- sich das Feedback-Element (12) aufgrund der von dem Feedback-Energieglied (14) bereitgestellten Energie so weit relativ zu der Kolbenstange (1) in der ersten axialen Richtung und über das Anschlagmerkmal (130) hinaus bewegt, bis das Feedback-Element (12) gegen das Aufprallmerkmal (201) stößt, wodurch ein haptisches und/oder hörbares Feedback erzeugt wird, **dadurch gekennzeichnet, dass**
- das Anschlagmerkmal (130) axial und/oder drehfest an der Kolbenstange (1) festgelegt ist.

2. Anordnung nach Anspruch 1, wobei
- die Kolbenstange (1) hohlzylindrisch ist,
- das Feedback-Element (12) im Anfangszustand vollständig innerhalb der Kolbenstange (1) angeordnet ist.

3. Anordnung nach einem der vorhergehenden Ansprüche, wobei
- das Aufprallmerkmal (201) bezüglich des Gehäuseelements (4) beweglich angeordnet ist,
- sich das Aufprallmerkmal (201) in dem freigegebenen Zustand bezüglich des Gehäuseelements (4) bewegt.

4. Anordnung nach einem der vorhergehenden Ansprüche, wobei
- das Anschlagmerkmal (130) axial und drehfest an der Kolbenstange (1) festgelegt ist,
- das Anschlagmerkmal (130) im Anfangszustand radial in den Hohlraum der Kolbenstange (1) hineinragt.

5. Anordnung nach einem der vorhergehenden Ansprüche, ferner aufweisend
- ein Energieglied (3), das dazu ausgestaltet ist, Energie bereitzustellen, um eine axiale Bewegung der Kolbenstange (1) in der distalen Richtung (D) zu induzieren, wobei
- im Anfangszustand
- die Kolbenstange (1) über eine Verriegelungsschnittstelle an das Gehäuseelement (4) gekoppelt ist, die eine durch das Energieglied (3) induzierte axiale Bewegung der Kolbenstange (1) verhindert,
- im freigegebenen Zustand
- die Verriegelungsschnittstelle freigegeben wird, so dass eine durch das Energieglied (3) induzierte axiale Bewegung der Kolbenstange (1) ermöglicht wird,
- sich die Kolbenstange (1) aufgrund der von dem Energieglied (3) bereitgestellten Energie in distaler Richtung (D) bewegt.

6. Anordnung nach Anspruch 5, ferner aufweisend
- ein bezüglich des Gehäuseelements (4) drehbar angeordnetes Transferglied (2),
- wobei das Transferglied (2) und die Kolbenstange (1) wirkgekoppelt sind, so dass eine Bewegung des Transferglieds (2) in einer ersten Drehrichtung in eine Bewegung der Kolbenstange (1) in distaler Richtung (D) umgewandelt wird,
- wobei im freigegebenen Zustand
- das Energieglied (3) ein Drehmoment auf das Transferglied (2) induziert, wodurch sich das Transferglied (2) in der ersten Drehrichtung dreht und dadurch die Kolbenstange (1) dazu zwingt, sich axial in distaler Richtung (D) zu bewegen.

7. Anordnung nach Anspruch 6, wobei
- das verschiebbare Anschlagmerkmal (130) im Anfangszustand durch das Transferglied (2) in der ersten radialen Position gehalten wird.

8. Anordnung nach Anspruch 7, wobei
- das Transferglied (2) eine Seitenwand aufweist, die das verschiebbare Anschlagmerkmal (130) in der ersten radialen Position hält, wenn die Anordnung im Anfangszustand ist,
- in der Seitenwand des Transferglieds (2) eine Aussparung (23) ausgebildet ist, wobei die Aussparung (23) in axialer Richtung auf das Anschlagmerkmal (130) ausgerichtet ist, wenn die Kolbenstange (1) in der Feedback-Position ist, so dass sich das verschiebbare Anschlagmerkmal (130) radial zu der Aussparung (23) hin oder in diese hinein verschieben kann.

9. Anordnung nach einem der Ansprüche 6 bis 8, wobei
- die Anordnung so ausgestaltet ist, dass sich das Transferglied (2) im freigegebenen Zustand axial bezüglich des Gehäuseelements (4) so weit bewegt, bis es an einem Endanschlag der Anordnung anschlägt.

10. Anordnung nach einem der vorhergehenden Ansprüche, wobei
- das Feedback-Energieglied (14) eine Feder ist,
- im Anfangszustand
- das Feedback-Energieglied (14) in dem Hohlraum der Kolbenstange (1) angeordnet ist und das Feedback-Element (12) in die erste axiale Richtung vorspannt,
- das Feedback-Element (12) in der ersten axialen Richtung an dem Anschlagmerkmal (130) anliegt.

11. Anordnung nach einem der vorhergehenden Ansprüche, wobei
- das Anschlagmerkmal (130) ein Vorsprung eines verschiebbaren Arms (13) ist,
- der verschiebbare Arm (13) axial ausgerichtet ist,
- das Anschlagmerkmal (130) im Bereich eines proximalen Endes der Kolbenstange (1) angeordnet ist,
- das Anschlagmerkmal (130) und/oder das Feedback-Element (12) ein Gleitmerkmal aufweisen, an dem das andere Element anliegen kann und an dem das andere Element zum Verschieben des Anschlagmerkmals (130) in radialer Richtung entlanggleiten kann.

12. Anordnung nach einem der vorhergehenden Ansprüche, wobei
- im Anfangszustand das verschiebbare Anschlagmerkmal (130), das in der ersten radialen Position ist, in einer radialen Richtung vorgespannt ist,
- sich das verschiebbare Anschlagmerkmal (130), wenn die Kolbenstange (1) die Feedback-Position erreicht, automatisch aus der ersten radialen Position in eine zweite radiale Position bewegt.

13. Anordnung nach einem der vorhergehenden Ansprüche, wobei
- im freigegebenen Zustand die Strecke, über die sich das Feedback-Element (12) durch das Feedback-Energieglied (14) induziert in der ersten axialen Richtung bewegt, mindestens dem Abstand zwischen der proximalen Position und der Feedback-Position der Kolbenstange (1) entspricht.

14. Anordnung nach einem der vorhergehenden Ansprüche, wobei
- das auf das Aufprallmerkmal (201) auftreffende Feedback-Element (12) ein hörbares Feedback von mindestens 20 dB erzeugt.

15. Medikamenten-Verabreichungsvorrichtung (1000), aufweisend:
- die Anordnung nach einem der vorhergehenden Ansprüche,
- ein Gehäuse (100), bei dem das Gehäuseelement (4) an dem Gehäuse (100) fixiert oder in diesem integriert ist,
- einen Medikamentenbehälter (8) mit einer Nadel (80), wobei der Medikamentenbehälter (8) ein Medikament aufweist,
- einen Nadelschutz (5), der teleskopisch an das Gehäuse (100) gekoppelt und bezüglich des Gehäuses (100) zwischen einer ausgezogenen Position, in der die Nadel (80) von dem Nadelschutz (5) abgedeckt ist, und einer zurückgezogenen Position, in der die Nadel (80) freiliegt, axial beweglich ist, wobei
- die Medikamenten-Verabreichungsvorrichtung (1000) dazu ausgestaltet ist, durch Bewegen des Nadelschutzes (5) aus der ausgezogenen Position in die zurückgezogene Position aus dem Anfangszustand in den freigegebenen Zustand umgeschaltet zu werden.

## Revendications

1. Agencement pour un dispositif d'administration de médicament (1000), comprenant :
- un élément de boîtier (4),
- une tige de piston (1) agencée mobile axialement par rapport à l'élément de boîtier (4),
- un élément de retour (12) agencé mobile axialement par rapport à l'élément de boîtier (4) et par rapport à la tige de piston (1),
- un organe d'énergie de retour (14) configuré pour fournir de l'énergie afin d'induire un déplacement de l'élément de retour (12) par rapport à la tige de piston (1) dans une première direction axiale,
- un élément de butée déplaçable radialement (130) et un élément d'impact (201),
- l'agencement présentant un état initial dans lequel
- la tige de piston (1) se trouve dans une position proximale,
- l'élément de retour (12) est agencé dans une cavité de la tige de piston (1),
- le déplacement de l'élément de retour (12) par rapport à la tige de piston (1) dans la première direction axiale est limité par l'élément de butée (130) se trouvant dans une première position radiale,
- l'agencement étant configuré pour être commuté de l'état initial vers un état libéré dans lequel
- la tige de piston (1) est déplacée dans une direction distale (D) au moins jusqu'à ce que la tige de piston (1) atteigne une position de retour,
- le déplacement de la tige de piston (1) vers la position de retour a pour effet de déplacer radialement l'élément de butée (130) pour permettre un déplacement de l'élément de retour (12) par rapport à la tige de piston (1) dans la première direction axiale au-delà de l'élément de butée (130),
- l'élément de retour (12) se déplace par rapport à la tige de piston (1) dans la première direction axiale et au-delà de l'élément de butée (130) sous l'effet de l'énergie fournie par l'organe d'énergie de retour (14) jusqu'à ce que l'élément de retour (12) heurte l'élément d'impact (201), produisant ainsi un retour tactile et/ou sonore, **caractérisé en ce que**
- l'élément de butée (130) est solidaire axialement et/ou en rotation de la tige de piston (1).

2. Agencement selon la revendication 1, dans lequel
- la tige de piston (1) est de forme cylindrique creuse,
- dans l'état initial, l'élément de retour (12) est entièrement agencé à l'intérieur de la tige de piston (1).

3. Agencement selon l'une quelconque des revendications précédentes, dans lequel
- l'élément d'impact (201) est agencé mobile par rapport à l'élément de boîtier (4), et
- dans l'état libéré, l'élément d'impact (201) se déplace par rapport à l'élément de boîtier (4).

4. Agencement selon l'une quelconque des revendications précédentes, dans lequel
- l'élément de butée (130) est solidaire axialement et en rotation de la tige de piston (1),
- dans l'état initial, l'élément de butée (130) fait saillie radialement dans la cavité de la tige de piston (1).

5. Agencement selon l'une quelconque des revendications précédentes, comprenant en outre
- un organe d'énergie (3) configuré pour fournir de l'énergie afin d'induire un déplacement axial de la tige de piston (1) dans la direction distale (D),
- dans l'état initial,
- la tige de piston (1) étant couplée à l'élément de boîtier (4) par l'intermédiaire d'une interface de verrouillage empêchant un déplacement axial de la tige de piston (1) induit par l'organe d'énergie (3),
- dans l'état libéré,
- l'interface de verrouillage étant libérée de sorte à autoriser un déplacement axial de la tige de piston (1) induit par l'organe d'énergie (3),
- la tige de piston (1) se déplaçant dans la direction distale (D) sous l'effet de l'énergie fournie par l'organe d'énergie (3).

6. Agencement selon la revendication 5, comprenant en outre
- un organe de transfert (2) agencé mobile en rotation par rapport à l'élément de boîtier (4),
- l'organe de transfert (2) et la tige de piston (1) étant couplés fonctionnellement de sorte qu'une rotation de l'organe de transfert (2) dans un premier sens de rotation est convertie en un déplacement de la tige de piston (1) dans la direction distale (D),
- dans l'état libéré,
- l'organe d'énergie (3) induisant un couple sur l'organe de transfert (2) sous l'effet duquel l'organe de transfert (2) effectue une rotation dans le premier sens de rotation et force ainsi la tige de piston (1) à se déplacer axialement dans la direction distale (D).

7. Agencement selon la revendication 6, dans lequel
- dans l'état initial, l'élément de butée déplaçable (130) est maintenu dans la première position radiale par l'organe de transfert (2).

8. Agencement selon la revendication 7, dans lequel
- l'organe de transfert (2) comprend une paroi latérale qui maintient l'élément de butée déplaçable (130) dans la première position radiale lorsque l'agencement présente l'état initial,
- un évidement (23) est formé dans la paroi latérale de l'organe de transfert (2), l'évidement (23) étant aligné dans la direction axiale avec l'élément de butée (130) lorsque la tige de piston (1) est dans la position de retour de sorte à permettre à l'élément de butée déplaçable (130) de se déplacer radialement vers ou dans l'évidement (23).

9. Agencement selon l'une quelconque des revendications 6 à 8,
- l'agencement étant configuré de sorte que, dans l'état libéré, l'organe de transfert (2) se déplace axialement par rapport à l'élément de boîtier (4) jusqu'à heurter une butée de fin de course de l'agencement.

10. Agencement selon l'une quelconque des revendications précédentes, dans lequel
- l'organe d'énergie de retour (14) est un ressort,
- dans l'état initial,
- l'organe d'énergie de retour (14) est agencé dans la cavité de la tige de piston (1) et sollicite l'élément de retour (12) dans la première direction axiale,
- l'élément de retour (12) vient en appui contre l'élément de butée (130) dans la première direction axiale.

11. Agencement selon l'une quelconque des revendications précédentes, dans lequel
- l'élément de butée (130) est une saillie d'un bras déplaçable (13),
- le bras déplaçable (13) est orienté axialement,
- l'élément de butée (130) est agencé au niveau d'une extrémité proximale de la tige de piston (1),
- l'un au moins parmi l'élément de butée (130) et l'élément de retour (12) comprend un élément de glissement contre lequel l'autre élément peut venir en appui et le long duquel l'autre élément peut glisser afin de déplacer l'élément de butée (130) dans la direction radiale.

12. Agencement selon l'une quelconque des revendications précédentes, dans lequel
- dans l'état initial, l'élément de butée déplaçable (130) se trouvant dans la première position radiale est sollicité dans une direction radiale,
- lorsque la tige de piston (1) atteint la position de retour, l'élément de butée déplaçable (130) passe automatiquement de la première position radiale à une deuxième position radiale.

13. Agencement selon l'une quelconque des revendications précédentes, dans lequel
- dans l'état libéré, la distance de déplacement de l'élément de retour (12) dans la première direction axiale induite par l'organe d'énergie de retour (14) est au moins égale à la distance entre la position proximale et la position de retour de la tige de piston (1).

14. Agencement selon l'une quelconque des revendications précédentes, dans lequel
- l'élément de retour (12), en heurtant l'élément d'impact (201), crée un retour audible d'au moins 20 dB.

15. Dispositif d'administration de médicament (1000), comprenant :
- l'agencement selon l'une quelconque des revendications précédentes,
- un boîtier (100), l'élément de boîtier (4) étant fixé au boîtier (100) ou y étant intégré,
- un contenant de médicament (8) pourvu d'une aiguille (80), le contenant de médicament (8) comprenant un médicament,
- un protège-aiguille (5) couplé télescopiquement au boîtier (100) et mobile axialement par rapport au boîtier (100) entre une position avancée, dans laquelle l'aiguille (80) est recouverte par le protège-aiguille (5), et une position rétractée, dans laquelle l'aiguille (80) est exposée,
- le dispositif d'administration de médicament (1000) étant configuré pour être commuté de l'état initial vers l'état libéré par déplacement du protège-aiguille (5) de la position avancée vers la position rétractée.
